# EUROPÄISCHE PATENTSCHRIFT

(11) **EP 3 923 850 B1**
(45) Veröffentlichungstag und Bekanntmachung des Hinweises auf die Patenterteilung: **26.04.2023**
(21) Anmeldenummer: 21709946.4
(22) Anmeldetag: 03.03.2021
(51) Int. Cl.: A61B 50/22, A61B 50/34, A61B 17/28, A61L 2/26

(54) **ENTSPERRVORRICHTUNG UND SIEBKORB**
UNLOCKING APPARATUS AND STRAINER BASKET
DISPOSITIF DE DÉVERROUILLAGE ET PANIER PERFORÉ

(30) Priorität: 06.03.2020 DE 102020106102
(43) Veröffentlichungstag der Anmeldung: 22.12.2021
(73) Patentinhaber: Aesculap AG, 78532 Tuttlingen (DE)
(72) Erfinder: HENKE, Matthias, 78048 Villingen-Schwenningen (DE); KIESSLING, Daniel, 78502 Villingen-Schwenningen (DE); LUZ, Sebastian, 78576 Emmingen-Liptingen (DE)
(74) Vertreter: Hoeger, Stellrecht & Partner Patentanwälte mbB
(86) Internationale Anmeldenummer: PCT/EP2021/055247
(87) Internationale Veröffentlichungsnummer: WO 2021/175883

(56) Entgegenhaltungen:
- WO-A1-2013/062925
- DE-A1-102017 102 027
- US-A- 4 229 420
- US-A- 5 449 069
- US-A1- 2011 114 522
- US-A1- 2014 216 966

## Beschreibung

Die vorliegende Erfindung betrifft eine Entsperrvorrichtung zum Entsperren von zusammenwirkende Sperrelemente umfassenden Sperreinrichtungen von mindestens zwei medizinischen Ringinstrumenten.

Ferner betrifft die vorliegende Erfindung einen Siebkorb mit einem Aufnahmeraum zur Aufnahme chirurgischer Instrumente.

In Siebkörben, auch als Grundsiebe bezeichnet, werden üblicherweise eine Mehrzahl an Instrumenten für chirurgische Eingriffe bereitgestellt. Die Instrumente müssen nach einer Operation wiederaufbereitet werden, und zwar unabhängig davon, ob sie bei der Operation genutzt wurden oder nicht. In der Regel ist ein Anteil an Ringinstrumenten mit Sperreinrichtungen, auch einfach als Sperre bezeichnet, in einem Grundsieb hoch. Bei den Ringinstrumenten kann es sich insbesondere um Nadelhalter und alle Arten von Klemmen handeln.

Um ein gutes Reinigungsergebnis erzielen zu können, müssen diese Ringinstrumente im geöffneten Zustand aufbereitet werden. Dies erfordert es bislang, bei jedem Instrument die Sperreinrichtung von Hand zu entsperren. Die zusammenwirkenden Sperrelemente der Sperreinrichtungen müssen dabei außer Eingriff gebracht werden, was auch als Ausrasten der Sperrelement bezeichnet wird. Nach dem Entsperren müssen die Instrumente noch geöffnet werden.

Das manuelle Entsperren und Öffnen der Ringinstrumente erhöht eine Vorbereitungszeit für einen Aufbereitungsprozess. Zudem kann die wiederholte Durchführung des beschriebenen Entsperrvorgangs bei einem Anwender Schmerzen in den Händen und Fingern verursachen.

Aus der US 2014/0216966 A1 sind ein einstellbarer Stringer für chirurgische Instrumente, ein Containersystem sowie ein Sterilisationsverfahren bekannt. Die WO 2013/062925 A1 offenbart ein Containersystem für chirurgische Instrumente und ein Sterilisationsverfahren. Ein Gestell für chirurgische Instrumente ist in der US 4,229,420 beschrieben. Eine Vorrichtung zum Aufbereiten und Bereitstellen chirurgischer Instrumente ist aus der US 5,449,069 bekannt. Die US 2011/0114522 A1 betrifft ein Containersystem für chirurgische Instrumente. In der DE 10 2017 102 027 A1 ist ein chirurgisches Instrument mit arretierbaren Schenkeln offenbart.

Es ist daher eine Aufgabe der vorliegenden Erfindung, eine Entsperrvorrichtung zum Entsperren von zusammenwirkende Sperrelemente umfassenden Sperreinrichtungen von mindestens zwei medizinischen Ringinstrumenten sowie einen Siebkorb bereitzustellen, die ein einfaches Entsperren von mindestens zwei medizinischen Ringinstrumenten ermöglichen.

Diese Aufgabe wird erfindungsgemäß gelöst durch eine Entsperrvorrichtung zum Entsperren von zusammenwirkende Sperrelemente umfassenden Sperreinrichtungen von mindestens zwei medizinischen Ringinstrumenten, wobei die zusammenwirkenden Sperrelemente in einer Sperrstellung kraft- und/oder formschlüssig in Eingriff und in einer Entsperrstellung außer Eingriff stehen, wobei die Ringinstrumente jeweils eine erste Branche mit einem ersten distalen Ende und einem ersten proximalen Ende und jeweils eine zweite Branche mit einem zweiten distalen Ende und einem zweiten proximalen Ende umfassen, wobei die erste Branche und die zweite Branche jedes Ringinstruments um eine Schwenkachse verschwenkbar aneinander gelagert sind, wobei am ersten proximalen Ende der ersten Branche jedes Ringinstruments ein erster Ring und wobei am zweiten proximalen Ende der zweiten Branche jedes Ringinstruments ein zweiter Ring angeordnet oder ausgebildet ist, wobei jeder erste Ring eine erste Ringebene definiert und wobei jeder zweite Ring eine zweite Ringebene definiert, wobei die erste Ringebene und die zweite Ringebene jedes Ringinstruments parallel oder im Wesentlichen parallel zueinander verlaufen, wobei die Schwenkachse quer, insbesondere senkrecht, zur ersten Ringebene und/oder zur zweiten Ringebene verläuft, wobei die Entsperrvorrichtung einen ersten Aufnahmekörper mit mindestens zwei Ringaufnahmen zum Aufnehmen der ersten Ringe der mindestens zwei Ringinstrumente umfasst, wobei die Entsperrvorrichtung einen Entsperrkörper mit mindestens zwei mit den zweiten Ringen der mindestens zwei Ringinstrumente zusammenwirkenden Entsperrelementen umfasst, wobei der Entsperrkörper und der Aufnahmekörper relativ zueinander in einer Betätigungsrichtung bewegbar angeordnet sind derart, dass dabei die mindestens zwei Entsperrelemente an den zweiten Ringen angreifen und diese relativ zu den ersten Ringen in einer Entsperrrichtung quer, insbesondere senkrecht, zur ersten Ringebene und zur zweiten Ringebene bewegen zum Überführen der Sperreinrichtungen der mindestens zwei Ringinstrumente von der Sperrstellung in die Entsperrstellung.

Mit einer solchen Entsperrvorrichtung ist es insbesondere möglich, zwei oder mehr, denkbar ist hier eine beliebige Anzahl, von Ringinstrumenten auf einfache Weise zu entsperren, und zwar durch Bewegen des Entsperrkörpers und des Aufnahmekörpers relativ zueinander in der Betätigungsrichtung. Durch diese Bewegung werden die zweiten Ringe relativ zu den ersten Ringen der Ringinstrumente in der beschriebenen Weise bewegt, wodurch die in der Sperrstellung in Eingriff stehenden Sperrelemente der Sperreinrichtungen außer Eingriff gebracht werden. Insbesondere ist es so möglich, zwei oder mehr Ringinstrumente gleichzeitig zu entsperren. Die Entsperrvorrichtung unterstützt also einen Anwender, der bislang die Ringinstrumente einzeln von Hand entsperren musste. Mit der Entsperrvorrichtung kann der Anwender insbesondere die ersten Ringe aller Ringinstrumente in jeweils eine der Ringaufnahmen des Aufnahmekörpers einlegen und dann mit dem Entsperrkörper durch Relativbewegung zum Aufnahmekörper in der Betätigungsrichtung die zweiten Ringe der Ringinstrumente relativ zu den ersten Ringen der Ringinstrumente derart bewegen, dass die Sperrelemente außer Eingriff gebracht und somit die Ringinstrumente von der Sperrstellung in die Entsperrstellung überführt werden. Anders als beim manuellen Entsperren von Ringinstrumenten, bei denen ein Anwender mit Daumen und Zeigefinger in die beiden Ringe des Ringinstruments eingreifen muss, um die Sperreinrichtung von der Sperrstellung in die Entsperrstellung zu überführen, muss der Anwender bei der vorgeschlagenen Entsperrvorrichtung lediglich den Aufnahmekörper und den Entsperrkörper relativ zueinander in der Betätigungsrichtung bewegen, um zwei oder mehr Ringinstrumente zu entsperren.

Günstig ist es, wenn der Aufnahmekörper und der Entsperrkörper zum Führen einer Bewegung relativ zueinander gekoppelt sind. Beispielsweise können sie über eine Nut-Feder-Verbindung verschieblich aneinander gehalten sein. So kann insbesondere die Betätigungsrichtung der Entsperrvorrichtung einem Anwender auf einfache Weise vorgegeben werden.

Günstig ist es, wenn die Entsperrvorrichtung eine Linearbewegungsführungseinrichtung und/oder eine Schwenkbewegungsführungseinrichtung umfasst. Die Linearbewegungsführungseinrichtung ermöglicht insbesondere das Führen einer linearen Bewegung des Aufnahmekörpers und des Entsperrkörpers relativ zueinander. Die Schwenkbewegungsführungseinrichtung ermöglicht insbesondere das Führen einer Schwenkbewegung um eine Schwenkachse des Aufnahmekörpers und des Entsperrkörpers relativ zueinander.

Eine Schwenkbewegung des Aufnahmekörpers und des Entsperrkörpers relativ zueinander kann insbesondere auf einfache Weise geführt werden, wenn die Schwenkbewegungsführungseinrichtung ein Schwenklager umfasst. Der Entsperrkörper und der Aufnahmekörper können insbesondere durch das Schwenklager miteinander beweglich gekoppelt sein. Das Schwenklager kann insbesondere Form eines Scharniergelenks ausgebildet sein.

Vorteilhaft ist es, wenn die Linearbewegungsführungseinrichtung mindestens ein geradlinig ausgebildetes Führungselement umfasst. Insbesondere kann das mindestens eine Führungselement in Form eines Führungsstabs ausgebildet sein. Die Linearbewegungsführungseinrichtung kann beispielsweise auch in Form einer Nut-Feder-Verbindung ausgebildet sein, um eine lineare oder geradlinige Bewegung des Aufnahmekörpers und des Entsperrkörpers relativ zueinander zu führen. Insbesondere können zwei oder mehr Führungselemente vorgesehen sein.

Vorzugsweise ist das mindestens eine Führungselement am Entsperrkörper oder am Aufnahmekörper angeordnet oder ausgebildet und in einer Führungselementaufnahme am anderen Körper verschieblich aufgenommen. Beispielsweise kann der Führungsstab am Entsperrkörper angeordnet sein, in eine Führungselementaufnahme am Aufnahmekörper eingreifen und in dieser verschiebbar ist. Entsprechend kann das Führungselement auch am Aufnahmekörper angeordnet oder ausgebildet sein und in einer Führungselementaufnahme am Entsperrkörper verschieblich aufgenommen werden.

Um möglichst viele Ringinstrumente mit der Entsperrvorrichtung entsperren zu können, ist es vorteilhaft, wenn der Aufnahmekörper eine Mehrzahl von Ringaufnahmen umfasst, wenn in jeder der Mehrzahl von Ringaufnahmen ein erster Ring eines Ringinstruments mindestens teilweise, insbesondere vollständig, einführbar ist und wenn der Entsperrkörper eine Mehrzahl von Entsperrelementen umfasst. Diese Ausgestaltung ermöglicht es insbesondere, eine Mehrzahl von Ringinstrumenten insbesondere gleichzeitig oder sukzessive mit der Entsperrvorrichtung durch einen Anwender zu entsperren.

Vorzugsweise entspricht eine Anzahl der Ringaufnahmen des Aufnahmekörpers einer Anzahl der Entsperrelemente des Entsperrkörpers. So ist sichergestellt, dass mit der Entsperrvorrichtung alle Ringinstrumente, die in die Ringaufnahmen des Aufnahmekörpers eingelegt werden können, auch bei einem Entsperrvorgang entsperrt werden können.

Um insbesondere eine Anzahl der Ringinstrumente schnell erfassen und überprüfen zu können, ist es vorteilhaft, wenn die mindestens zwei Ringaufnahmen des Aufnahmekörpers und/oder die mindestens zwei Entsperrelemente fortlaufend nummeriert sind. So kann ein Anwender, beispielsweise dann, wenn die Entsperrvorrichtung in einem Siebkorb aufgenommen ist, schnell erfassen, ob alle Instrumente vollzählig vorhanden sind.

Die Entsperrvorrichtung lässt sich auf einfache Weise ausbilden, wenn jede der mindestens zwei Ringaufnahmen in Form eines Aufnahmeschlitzes oder einer Aufnahmenut ausgebildet ist.

Gemäß einer weiteren bevorzugten Ausführungsform der Erfindung kann vorgesehen sein, dass jede der mindestens zwei Ringaufnahmen eine Widerlagerfläche für einen aufgenommenen ersten Ring aufweist und dass die Widerlagerfläche parallel oder im Wesentlichen parallel zur ersten Ringebene verläuft. Beim Entsperren, also bei einer Bewegung des Entsperrkörpers und des Aufnahmekörpers relativ zueinander in der Betätigungsrichtung, liegen die ersten Ringe der Ringinstrumente an den Widerlagerflächen der Ringaufnahmen an, so dass mit den Entsperrelementen die zweiten Ringe der Ringinstrumente relativ zu den ersten Ringen in der beschriebenen Weise bewegt werden können, um die Sperreinrichtungen der Ringinstrumente zu entsperren.

Ferner ist es vorteilhaft, wenn die mindestens zwei Entsperrelemente jeweils eine Entsperrfläche aufweisen, an denen die zweiten Ringe bei einer Bewegung des Entsperrkörpers und des Aufnahmekörpers relativ zueinander in der Betätigungsrichtung anliegen und/oder aufgleiten. Diese Ausgestaltung der mindestens zwei Entsperrelemente ermöglicht ein einfaches Zusammenwirken derselben mit den zweiten Ringen, um die Sperreinrichtungen der Ringinstrumente zu entsperren. Die Entsperrflächen können insbesondere als Anlageflächen und/oder als Aufgleitflächen, die auch als Anlaufschrägen bezeichnet werden können, ausgebildet sein.

Günstig ist es, wenn jede Widerlagerfläche eine Widerlagerebene definiert, wenn jede Entsperrfläche eine Entsperrebene definiert und wenn ein Abstand zwischen der Widerlagerebene und der Entsperrebene von Ringaufnahmen und Entsperrelementen, die einander zugeordnet sind zum Lösen der Sperreinrichtung eines der mindestens zwei Ringinstrumente, beim Bewegen des Entsperrkörpers und des Aufnahmekörpers relativ zueinander in der Betätigungsrichtung abnimmt. Mit anderen Worten können durch die Verringerung des Abstands der Widerlagerebene und der Entsperrebene bei der Bewegung des Entsperrkörpers und des Aufnahmekörpers relativ zueinander die ersten und zweiten Ringe jedes Ringinstruments relativ zueinander parallel verschoben werden, wodurch sich die Sperreinrichtungen der Ringinstrumente auf einfache Weise von der Sperrstellung in die Entsperrstellung überführen lassen.

Günstig ist es, wenn der Aufnahmekörper eine Längsdurchbrechung aufweist und wenn die Längsdurchbrechung die mindestens zwei Ringaufnahmen durchsetzt. Diese Ausgestaltung ermöglicht es insbesondere, beispielsweise ein Halteglied in die Längsdurchbrechung einzuführen, wenn die Ringinstrumente mit ihren ersten Ringen in den Ringaufnahmen des Aufnahmekörpers aufgenommen sind. So können alle ersten Ringe auf einfache Weise auf dem Halteglied aufgefädelt werden.

Auf einfache Weise lässt sich der Aufnahmekörper ausbilden, wenn die Längsdurchbrechung in Form einer Bohrung ausgebildet ist. Sie kann auch in Form einer Nut oder eines Schlitzes am Aufnahmekörper ausgebildet sein.

Um die ersten Ringe der Ringinstrumente in der beschriebenen Weise gemeinsam handhaben zu können, ist es vorteilhaft, wenn die Entsperrvorrichtung ein Halteglied umfasst, welches in einer Haltestellung in der Längsdurchbrechung aufgenommen ist. Sind die ersten Ringe der Ringinstrumente bereits in die Ringaufnahmen eingesetzt, können so alle ersten Ringe auf das Halteglied aufgefädelt werden. Die Entsperrvorrichtung kann optional auch ein weiteres Halteglied umfassen, auf das die zweiten Ringe der Ringinstrumente aufgefädelt werden können. Beide Halteglieder können auch miteinander gekoppelt sein. Beispielsweise kann so eine U-förmige Anordnung ausgebildet werden, bei der die beiden Halteglieder die Schenkel der Anordnung bilden, auf denen die ersten und zweiten Ringe aller Ringinstrumente aufgefädelt sind. Mit einer solchen Anordnung, die auch als Stringer bezeichnet wird, können die Ringinstrumente definiert in einer geöffneten Stellung gehalten werden. Ein Abstand der Halteglieder definiert dabei, wie weit die Ringinstrumente geöffnet sind.

Günstigerweise ist das Halteglied stabförmig ausgebildet. Ein solches Halteglied lässt sich auf einfache Weise herstellen und handhaben.

Um die Herstellung der Entsperrvorrichtung so einfach wie möglich zu gestalten, ist es vorteilhaft, wenn der Aufnahmekörper quaderförmig oder im Wesentlichen quaderförmig ausgebildet ist und eine Längsrichtung definiert und wenn die Längsrichtung quer, insbesondere senkrecht, zu den Ringaufnahmen verläuft. Quaderförmig bedeutet in diesem Sinne insbesondere eine Grundform oder ursprüngliche Form des Aufnahmekörpers, welcher zur Ausbildung der Ringaufnahmen und insbesondere auch der Längsdurchbrechung derart bearbeitet sein kann, dass er in einem Querschnitt quer zur Längsrichtung im Wesentlichen eine L-Form oder eine U-Form aufweist.

Das Entsperren der Ringinstrumente kann insbesondere dadurch vereinfacht werden, dass die Längsrichtung quer zu den Widerlagerflächen verläuft.

Die Ringe der Ringinstrumente lassen sich insbesondere auf einfache Weise auf ein Halteglied auffädeln, wenn sich die Längsdurchbrechung parallel zur Längsrichtung erstreckt.

Vorteilhaft ist es, wenn die Betätigungsrichtung parallel zur Längsrichtung verläuft. Zum Entsperren der Ringinstrumente müssen also der Aufnahmekörper und der Entsperrkörper relativ zueinander lediglich parallel zur Längsrichtung des Aufnahmekörpers bewegt werden.

Gemäß einer weiteren bevorzugten Ausführungsform der Erfindung kann vorgesehen sein, dass der Aufnahmekörper und der Entsperrkörper in einer Richtung quer, insbesondere senkrecht, zu den mindestens zwei Ringaufnahmen, insbesondere quer zu den ersten Ringebenen der mindestens zwei Ringinstrumente, bewegbar sind zum Überführen der Sperreinrichtungen der mindestens zwei Ringinstrumente von der Sperrstellung in die Entsperrstellung. Mit anderen Worten werden bei dieser Ausführungsform der Aufnahmekörper und der Entsperrkörper in einer Richtung quer zu den Ringaufnahmen relativ zueinander bewegt. Insbesondere kann es sich dabei um die Längsrichtung des Aufnahmekörpers handeln.

Der Aufbau der Entsperrvorrichtung kann insbesondere dadurch vereinfacht werden, dass der der Aufnahmekörper und der Entsperrkörper identisch ausgebildet sind. Mit anderen Worten umfasst die Entsperrvorrichtung zwei der oben beschriebenen bevorzugten Ausführungsformen von Aufnahmekörpern. Der eine Aufnahmekörper nimmt in seinen Ringaufnahmen die ersten Ringe der Ringinstrumente auf. Der zweite Aufnahmekörper die zweiten Ringe der Ringinstrumente. Zum Entsperren der Ringinstrumente können dann die beiden Aufnahmekörper parallel zu den von ihnen definierten Längsrichtungen relativ zueinander bewegt werden.

Günstig ist es, wenn die Widerlagerflächen des identisch zum Aufnahmekörper ausgebildeten Entsperrkörpers die Entsperrflächen definieren. Dies ermöglicht es auf einfache Weise, die erforderlichen Kräfte zum Entsperren der Sperreinrichtungen der Ringinstrumente aufzubringen.

Ferner ist es günstig, wenn die Ringaufnahmen des Aufnahmekörpers und die Ringaufnahmen des identisch zum Aufnahmekörper ausgebildeten Entsperrkörpers einander spiegelsymmetrisch gegenüberliegend angeordnet sind zum Aufnehmen einerseits der ersten Ringe und andererseits der zweiten Ringe der mindestens zwei Ringinstrumente. Diese Anordnung der beiden identischen Aufnahmekörper ermöglicht ein einfaches Einsetzen der Ringinstrumente mit ihren Ringen in die jeweiligen Ringaufnahmen.

Vorzugsweise erstrecken sich die Ringaufnahmen von einer Aufnahmekörperoberseite zu einer Aufnahmekörperunterseite. Diese Ausgestaltung hat insbesondere den Vorteil, dass die Entsperrvorrichtung leicht reinigbar ist. Die Ringaufnahmen lassen sich so vollständig durchspülen. Insbesondere behindern sie dadurch auch eine Reinigung der in der Entsperrvorrichtung aufgenommenen Ringinstrumente nicht, beispielsweise wenn diese in einer Waschmaschine gewaschen und dann anschließend in einer Heißdampfsterilisationsvorrichtung heißdampfsterilisiert werden.

Gemäß einer weiteren bevorzugten Ausführungsform der Erfindung kann vorgesehen sein, dass der Aufnahmekörper und der Entsperrkörper zum Öffnen der Ringinstrumente nach dem Überführen von der Sperrstellung in die Entsperrstellung durch Verschwenken der ersten und zweiten Ringe voneinander weg in einer Öffnungsrichtung bewegbar sind, die quer, insbesondere senkrecht, zur Betätigungsrichtung verläuft. Mit anderen Worten können der Aufnahmekörper und der Entsperrkörper in der Öffnungsrichtung voneinander weg bewegt werden. Bei dieser Bewegung werden die Ringinstrumente geöffnet durch Verschwenken ihrer beiden Branchen relativ zueinander um die Schwenkachsen der Ringinstrumente. Mit der Entsperrvorrichtung lassen sich also nicht nur zwei oder mehr Instrumente auf einfache Weise entsperren, sondern auch öffnen, was für eine optimale Reinigung und Aufbereitung der Ringinstrumente vorteilhaft ist.

Gemäß einer weiteren bevorzugten Ausführungsform der Erfindung kann vorgesehen sein, dass der Aufnahmekörper und der Entsperrkörper in der Betätigungsrichtung parallel oder im Wesentlichen parallel zu den mindestens zwei Ringaufnahmen, insbesondere parallel zu den ersten Ringebenen der mindestens zwei Ringinstrumente, bewegbar sind zum Überführen der Sperreinrichtungen der mindestens zwei Ringinstrumente von der Sperrstellung in die Entsperrstellung. Eine solche Bewegung kann insbesondere dadurch realisiert werden, dass der Aufnahmekörper und der Entsperrkörper zunächst etwas voneinander entfernt sind und dann zum Entsperren der Ringinstrumente aufeinander zu bewegt werden. Zum Entsperren der Ringinstrumente können also die ersten Ringe beispielsweise in die Ringaufnahmen des Aufnahmekörpers eingelegt werden, nachdem zunächst der Entsperrkörper vom Aufnahmekörper weg bewegt wurde. Zum Entsperren der Ringinstrumente wird dann der Entsperrkörper in Richtung auf den Aufnahmekörper hin bewegt. Dabei wirken dann die Entsperrelemente des Entsperrkörpers in der beschriebenen Weise mit den zweiten Ringen der Ringinstrumente zusammen, um diese relativ zu den ersten Ringen in der Entsperrrichtung zu bewegen zum Überführen der Sperreinrichtungen der Ringinstrumente von der Sperrstellung in die Entsperrstellung.

Günstig ist es, wenn die Betätigungsrichtung in einer Betätigungsebene verläuft, die sich parallel zu den ersten und/oder zweiten Ringebenen erstreckt. Die Betätigungsrichtung muss nicht zwingend linear sein. Sie kann auch eine gekrümmte Bahn definieren.

Günstig ist es, wenn der Aufnahmekörper und der Entsperrkörper relativ zueinander in der Betätigungsebene verschiebbar und/oder um eine quer, insbesondere senkrecht, zu den ersten und/oder zweiten Ringebenen verlaufene Schwenkachse verschwenkbar angeordnet oder ausgebildet sind. Diese Ausgestaltung ermöglicht es insbesondere, den Aufnahmekörper und den Entsperrkörper relativ zueinander längs einer geradlinigen Bahn, längs einer Kreisbahn oder durch eine Überlagerung einer geradlinigen Bewegung und einer Schwenkbewegung zu bewegen, um die Ringinstrumente zu entsperren.

Die Entsperrvorrichtung kann insbesondere auf einfache Weise ausgebildet werden, wenn die Betätigungsrichtung quer, insbesondere senkrecht, zur Längsrichtung des Aufnahmekörpers verläuft.

Vorteilhaft ist es, wenn die zweiten Ringe bei einer Bewegung des Entsperrkörpers und des Aufnahmekörpers relativ zueinander in der Betätigungsrichtung jeweils an einer Entsperrfläche des Entsperrkörpers anliegen und aufgleiten. Diese Ausgestaltung ermöglicht es insbesondere, die zweiten Ringe der Ringinstrumente in definierter Weise zu bewegen. Eine Bewegung in der Entsperrrichtung kann also insbesondere davon abhängen, wie weit der Entsperrkörper und der Aufnahmekörper bereits aufeinander zu bewegt sind.

Günstigerweise sind von den Entsperrflächen definierte Entsperrebenen relativ zu den zugeordneten Widerlagerebenen um einen Neigungswinkel geneigt. Diese Ausgestaltung ermöglicht insbesondere auf einfache Weise das Aufgleiten der zweiten Ringe an den Entsperrflächen bei einer Bewegung des Entsperrkörpers und des Aufnahmekörpers in der Betätigungsrichtung aufeinander zu. Durch diese Ausgestaltung der Betätigungselemente kann also eine Betätigungskraft, die von einem Anwender in der Betätigungsrichtung aufgebracht wird, in die Entsperrrichtung umgelenkt werden, um die ersten Ringe und die zweiten Ringe in der Entsperrrichtung relativ zueinander zu bewegen zum Überführen der Sperreinrichtungen von der Sperrstellung in die Entsperrstellung.

Die Sperreinrichtungen der Ringinstrumente können insbesondere auf einfache Weise entsperrt werden, wenn der Neigungswinkel in einem Bereich von etwa 10° bis etwa 50° liegt.

Vorzugsweise ist der Neigungswinkel aller Entsperrflächen identisch. Diese Ausgestaltung ermöglicht es insbesondere, die Entsperrvorrichtung derart auszubilden, dass alle Ringinstrumente gleichzeitig entsperrt werden können.

Ferner kann es vorteilhaft sein, wenn sich die Neigungswinkel der Entsperrflächen des Entsperrkörpers unterscheiden. Dadurch kann insbesondere erreicht werden, dass die Ringinstrumente nicht gleichzeitig, sondern nacheinander, also sukzessive entsperrt werden. Je größer der Neigungswinkel ist, umso eher werden die Ringinstrumente entsperrt. Bei einer Bewegung des Entsperrkörpers und des Aufnahmekörpers aufeinander zu werden also zunächst diejenigen Ringinstrumente entsperrt, die mit Entsperrelementen zusammenwirken, deren Neigungswinkel der Entsperrflächen am größten sind.

Vorzugsweise nehmen die Neigungswinke benachbarter Entsperrflächen sukzessive zu. So können benachbart in der Entsperrvorrichtung aufgenommene Ringinstrumente nacheinander geöffnet werden, und zwar durch eine einmalige Bewegung des Entsperrkörpers und des Aufnahmekörpers aufeinander zu. Insbesondere können die Neigungswinkel benachbarter Entsperrflächen um einen Änderungswinkel in einem Bereich von etwa 1° bis etwa 10° zunehmen. Insbesondere kann der Änderungswinkel etwa 2° bis etwa 3° betragen. Ferner kann der Änderungswinkel auch von einer Anzahl der Ringaufnahmen am Aufnahmekörper abhängen. Je größer die Anzahl der Ringaufnahmen ist, umso kleiner der Änderungswinkel. So kann sichergestellt werden, dass alle Ringinstrumente mit der Entsperrvorrichtung auf einfache Weise entsperrt werden können.

Überdies kann es vorteilhaft sein, wenn ein Abstand von in Richtung auf den Aufnahmekörper hin weisenden Enden der Entsperrflächen von einer Entsperrkörperunterseite, die in Richtung auf den Aufnahmekörper hin weist, identisch sind. Diese Ausgestaltung ermöglicht es insbesondere, dass alle Entsperrelemente gleichzeitig mit zweiten Ringen der Ringinstrumente in Kontakt treten und mit diesen zum Entsperren der Sperreinrichtungen zusammenwirken können. Dies ist insbesondere unabhängig davon, ob die Neigungswinkel aller Entsperrflächen identisch sind oder sich unterscheiden. Sind in diesem Fall die Neigungswinkel identisch, können die Ringinstrumente gleichzeitig entsperrt werden. Sind die Neigungswinkel der Entsperrflächen unterschiedlich, können die Ringinstrumente in der beschriebenen Weise sukzessive entsperrt werden.

Ferner kann es günstig sein, wenn ein Abstand von in Richtung auf den Aufnahmekörper hin weisenden Enden der Entsperrflächen von einer Entsperrkörperunterseite, die in Richtung auf den Aufnahmekörper hin weist, unterschiedlich ist. Diese Ausgestaltung hat insbesondere zur Folge, dass bei einer Bewegung des Entsperrkörpers und des Aufnahmekörpers aufeinander zu die zweiten Ringe der Ringinstrumente nicht gleichzeitig, sondern sukzessive, also nacheinander, mit den Entsperrflächen der Entsperrelemente in Kontakt treten und zum Entsperren der Sperreinrichtungen zusammenwirken können. So können insbesondere dann, wenn die Neigungswinkel aller Entsperrflächen identisch sind, die Ringinstrumente trotzdem nicht gleichzeitig, sondern sukzessive entsperrt werden.

Um ein sukzessives Entsperren der Mehrzahl von Ringelementen mit der Entsperrvorrichtung zu ermöglichen, ist es günstig, wenn der Abstand benachbarter Entsperrflächen von der Entsperrkörperunterseite sukzessive zunimmt. Insbesondere kann der Abstand linear zunehmen.

Gemäß einer weiteren bevorzugten Ausführungsform der Erfindung kann vorgesehen sein, dass der Aufnahmekörper eine Kopplungseinrichtung umfasst zum kraft- und/oder formschlüssigen Koppeln mit einem Siebkorb in einer Kopplungsstellung. Die Kopplungseinrichtung ermöglicht insbesondere eine definierte und sichere Anordnung des Aufnahmekörpers im Siebkorb. Insbesondere kann er so unbeweglich mit dem Siebkorb gekoppelt werden. Zur Handhabung der Entsperrvorrichtung kann dann in diesem Fall ein Anwender mit einer Hand den Entsperrkörper bewegen und mit der anderen Hand den Siebkorb halten. Er muss also nicht direkt am Aufnahmekörper angreifen, um die Ringinstrumente zu entsperren.

Gemäß einer weiteren bevorzugten Ausführungsform der Erfindung kann vorgesehen sein, dass die Entsperrvorrichtung ausgebildet ist zum gleichzeitigen Lösen aller Sperreinrichtungen der in der Entsperrvorrichtung aufgenommenen Ringinstrumente bei einer Bewegung des Entsperrkörpers und des Aufnahmekörpers relativ zueinander in der Betätigungsrichtung. Das gleichzeitige Lösen oder Entsperren aller Sperreinrichtungen ist mit unterschiedlichen Ausführungsformen, die oben bereits beschrieben wurden, möglich.

Ferner kann es vorteilhaft sein, wenn die Entsperrvorrichtung ausgebildet ist zum sukzessiven Lösen aller Sperreinrichtungen der in der Entsperrvorrichtung aufgenommenen Instrumente bei einer Bewegung des Entsperrkörpers und des Aufnahmekörpers relativ zueinander in der Betätigungsrichtung. Insbesondere kann dies erreicht werden durch Variation eines Abstands von in Richtung auf den Aufnahmekörper hin weisenden Enden der Entsperrflächen von einer Entsperrkörperunterseite, wie dies bereits oben beschrieben wurde, oder durch Variation des Neigungswinkels der Entsperrflächen des Entsperrkörpers. Dies wurde oben ebenfalls eingehend erläutert.

Damit die Entsperrvorrichtung bei einer Aufbereitung der Ringinstrumente in einem Siebkorb verbleiben kann, ist es vorteilhaft, wenn die Entsperrvorrichtung aus einem oder mehreren Werkstoffen ausgebildet ist, die heißdampfsterilisierbar sind. Insbesondere kann es sich bei den Werkstoffen um Kunststoffe oder Metalle handeln, beispielsweise Polyetheretherketon (PEEK) oder einen Instrumentenstahl. Insbesondere lassen sich der Aufnahmekörper und der Entsperrkörper auf einfache Weise aus einem Kunststoff ausbilden. Die beschriebenen Halteglieder und das mindestens einen Führungselements können insbesondere aus einem metallischen Werkstoff ausgebildet sein, um eine Stabilität der Entsperrvorrichtung zu verbessern.

Günstig ist es, wenn am Aufnahmekörper und/oder am Entsperrkörper ein Griffelement angeordnet ist zum Handhaben der Entsperrvorrichtung. Ein Griffelement kann insbesondere in Form eines abstehenden Bügels ausgebildet sein. Griffelemente lassen sich auch ausbilden durch ergonomische Formgebung des Aufnahmekörpers und des Entsperrkörpers, beispielsweise durch Vorsehen von Griffmulden für einzelne oder mehrere Finger einer Hand eines Anwenders. Ein Griffelement kann insbesondere auch ausgebildet sein in Form eines Betätigungshebels, welcher mit dem Entsperrkörper starr oder beweglich gekoppelt ist, um diesen relativ zum Aufnahmekörper zu bewegen. Ein solcher Betätigungshebel kann insbesondere in Form eines Exzenterhebels ausgebildet sein, um einen Kraftaufwand für einen Anwender zum Betätigen der Entsperrvorrichtung zum Entsperren der in dieser aufgenommenen Ringinstrumente zu minimieren.

Die eingangs gestellte Aufgabe wird ferner bei einem Siebkorb der eingangs beschriebenen Art erfindungsgemäß dadurch gelöst, dass im Aufnahmeraum eine der oben beschriebenen Entsperrvorrichtungen angeordnet oder ausgebildet ist.

Ein solcher Siebkorb weist insbesondere die bereits oben im Zusammenhang mit bevorzugten Ausführungsformen von Entsperrvorrichtungen beschriebenen Vorteile auf. Weiter ist es beispielsweise günstig, eine Entsperrvorrichtung im Aufnahmeraum anzuordnen, um eine Übersichtlichkeit der im Siebkorb aufgenommenen Instrumente zu erhöhen. Die Instrumente können so insbesondere diskret geordnet im Siebkorb angeordnet werden. Dies ermöglicht zudem eine einfache Überprüfung der Vollständigkeit des Siebkorbs, also insbesondere ob alle bei einem chirurgischen Eingriff eingesetzten Instrumente wieder vollständig im Siebkorb enthalten sind. Überdies kann das Entsperren der Ringinstrumente auf einfache Weise im Siebkorb durchgeführt werden.

Vorteilhaft ist es, wenn der Siebkorb einen Siebkorbboden und sich vom Siebkorbboden quer, insbesondere senkrecht, weg erstreckende Siebkorbwände umfasst, wenn der Siebkorbboden und die Siebkorbwände den Aufnahmeraum begrenzen und wenn der Siebkorbboden und/oder die Siebkorbwände mit einer Mehrzahl von Perforationen versehen sind. Perforationen in diesem Sinne können insbesondere Durchbrechungen jedweder Art sein. Deren Form und Größe kann identisch sein oder variieren. Beispielsweise können der Siebkorbboden und die Siebkorbwände plattenförmig ausgebildet sein und mit im Querschnitt kreisförmigen oder vieleckigen Durchbrechungen versehen sein. Die Perforationen lassen sich insbesondere nutzen, um insbesondere den Aufnahmekörper mit dem Siebkorb temporär oder dauerhaft zu koppeln.

Günstiger Weise ist die Kopplungseinrichtung des Aufnahmekörpers ausgebildet zum kraft- und/oder formschlüssigen in Eingriff bringen mit Perforationen des Siebkorbbodens und/oder der Siebkorbwände in der Kopplungsstellung. Beispielsweise können am Aufnahmekörper klammerförmige Vorsprünge ausgebildet sein, die in die Perforationen eingehakt werden. Denkbar sind auch elastische Vorsprünge aus einem Kunststoff, die in der Kopplungsstellung klemmend in den Perforationen gehalten werden.

Ferner wird die Verwendung einer der oben beschriebenen Sperrvorrichtungen vorgeschlagen zum gleichzeitigen oder sukzessiven außer Eingriff Bringen miteinander zusammenwirkender Sperrelemente von Sperreinrichtungen von mindestens zwei medizinischen Ringinstrumenten.

Wie bereits beschrieben können hierfür erste Ringe der Ringinstrumente in die Ringaufnahmen des Aufnahmekörpers eingeführt und dann zum Entsperren der Sperreinrichtungen der Entsperrkörper relativ zum Aufnahmekörper in der Betätigungsrichtung bewegt werden.

Die nachfolgende Beschreibung bevorzugter Ausführungsformen der Erfindung die in dem Zusammenhang mit den Zeichnungen der näheren Erläuterung. Es zeigen:
- Figur 1:: eine perspektivische Gesamtansicht eines Ausführungsbeispiels Siebkorbs mit zwei Ausführungsbeispielen von im Siebkorb aufgenommenen Entsperrvorrichtungen mit eingelegten Ringinstrumenten;
- Figur 2:: eine Draufsicht auf ein Ausführungsbeispiel einer Entsperrvorrichtung mit aufgenommenen Ringinstrumenten;
- Figur 3:: eine Schnittansicht längs Linie 3-3 in Figur 2;
- Figur 4:: eine perspektivische Ansicht der Entsperrvorrichtung aus den Figuren 2 und 3;
- Figur 5:: eine Ansicht der Entsperrvorrichtung ähnlich Figur 2, wobei der Aufnahmekörper und der Entsperrkörper relativ zueinander etwas in der Betätigungsrichtung bewegt sind;
- Figur 6:: eine perspektivische Ansicht der Anordnung aus Figur 5;
- Figur 7:: eine vergrößerte Ausschnittansicht des Bereichs A aus Figur 6;
- Figur 8:: eine perspektivische Ansicht der Entsperrvorrichtung aus den Figuren 2 bis 7 mit geöffneten Ringinstrumenten;
- Figur 9:: eine Draufsicht auf die Anordnung aus Figur 8 von oben;
- Figur 10:: eine perspektivische Ansicht eines Aufnahmekörpers einer Entsperrvorrichtung;
- Figur 11:: eine Ansicht der Anordnung aus Figur 10 in Richtung des Pfeils B;
- Figur 12:: eine Ansicht der Anordnung aus Figur 10 in Richtung des Pfeils C;
- Figur 13:: eine Schnittansicht längs Linie 13-13 in Figur 12;
- Figur 14:: eine Schnittansicht längs Linie 14-14 in Figur 12;
- Figur 15:: eine perspektivische Gesamtansicht eines weiteren Ausführungsbeispiels einer Entsperrvorrichtung mit in dieser aufgenommenen Ringinstrumenten;
- Figur 16:: eine Ansicht der Anordnung aus Figur 15 in Richtung des Pfeils D von oben;
- Figur 17:: eine Ansicht der Anordnung aus Figur 16 in Richtung des Pfeils E von vorn;
- Figur 18:: eine Ansicht ähnlich Figur 15, wobei der Entsperrkörper etwas auf den Aufnahmekörper zum Entsperren der Ringinstrumente hin bewegt ist;
- Figur 19:: eine vergrößerte Teilansicht des Bereichs F aus Figur 18;
- Figur 20:: eine Ansicht der Anordnung aus Figur 18, wobei mit der Entsperrkörper und der Aufnahmekörper noch weiter aneinander angenähert sind;
- Figur 21:: eine Ansicht der Anordnung aus Figur 18 in Richtung des Pfeils G von vorn;
- Figur 22:: eine Ansicht der Anordnung aus Figur 15 mit nach dem Entsperren geöffneten Ringinstrumenten;
- Figur 23:: eine schematische perspektivische Gesamtansicht eines weiteren Ausführungsbeispiels einer Entsperrvorrichtung mit aufgenommenen Ringinstrumenten vor dem Entsperren;
- Figur 24:: eine weitere perspektivische Ansicht der Anordnung aus Figur 23;
- Figur 25:: eine schematische Ansicht der Anordnung aus den Figuren 23 und 24 beim Entsperren der Ringinstrumente;
- Figur 26:: eine schematische Ansicht eines weiteren Ausführungsbeispiels einer Entsperrvorrichtung; und
- Figur 27:: eine schematische Ansicht eines weiteren Ausführungsbeispiels einer Entsperrvorrichtung.

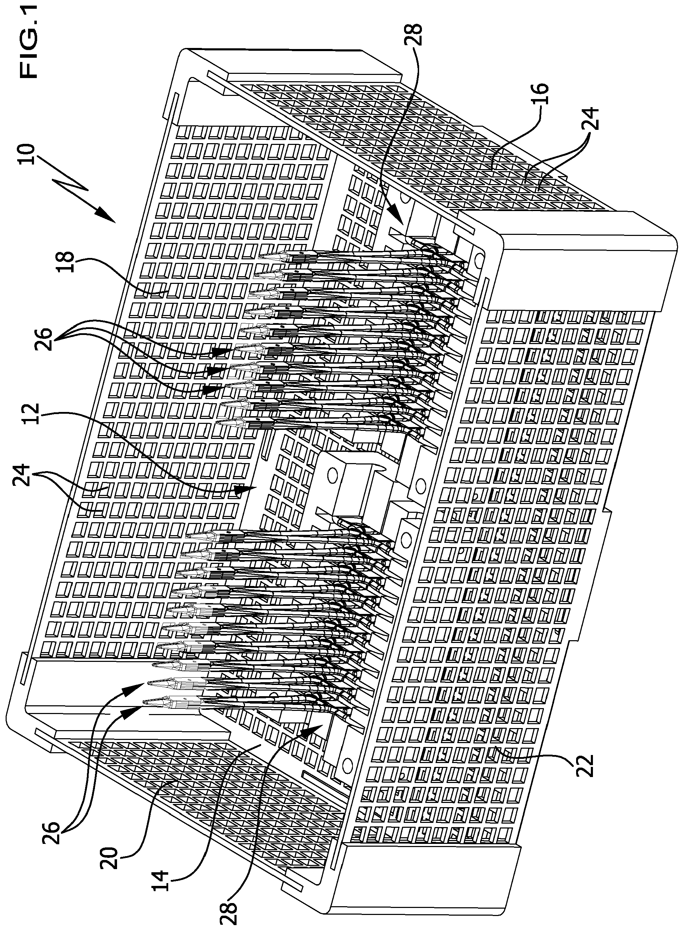

In Figur 1 ist ein Ausführungsbeispiel eines Siebkorbs 10 mit einem Aufnahmeraum 12 schematisch dargestellt.

Der Siebkorb 10 umfasst einen Siebkorbboden 14 und sich vom Siebkorbboden 14 quer weg erstreckende Siebkorbwände 16, 18, 20 und 22. Der Siebkorbboden 14 und die Siebkorbwände 16, 18, 20 und 22 begrenzen den Aufnahmeraum 12.

Die Siebkorbwände 16 und 20 sind bei dem in Figur 1 dargestellten Ausführungsbeispiel des Siebkorbs 10 parallel zueinander ausgerichtet. Die Siebkorbwände 18 und 22 sind ebenfalls parallel zueinander ausgerichtet.

Der Siebkorbboden 14 und die Siebkorbwände 16, 18, 20 und 22 sind jeweils mit einer Mehrzahl von Perforationen 24 versehen. Diese sind in Form quadratischer Durchbrechungen ausgebildet und in einem regelmäßigen Muster, nämlich einem zweidimensionalen Raster, angeordnet.

Der Siebkorb 10 dient zum Aufnehmen chirurgischer Instrumente, beispielsweise den in Figur 1 schematisch und rein beispielhaft dargestellten Ringinstrumenten 26. Die Ringinstrumente 26 sind in Form von Nadelhaltern ausgebildet. Sie können alternativ auch in Form von Klemmen oder anderer Instrumente ausgebildet sein.

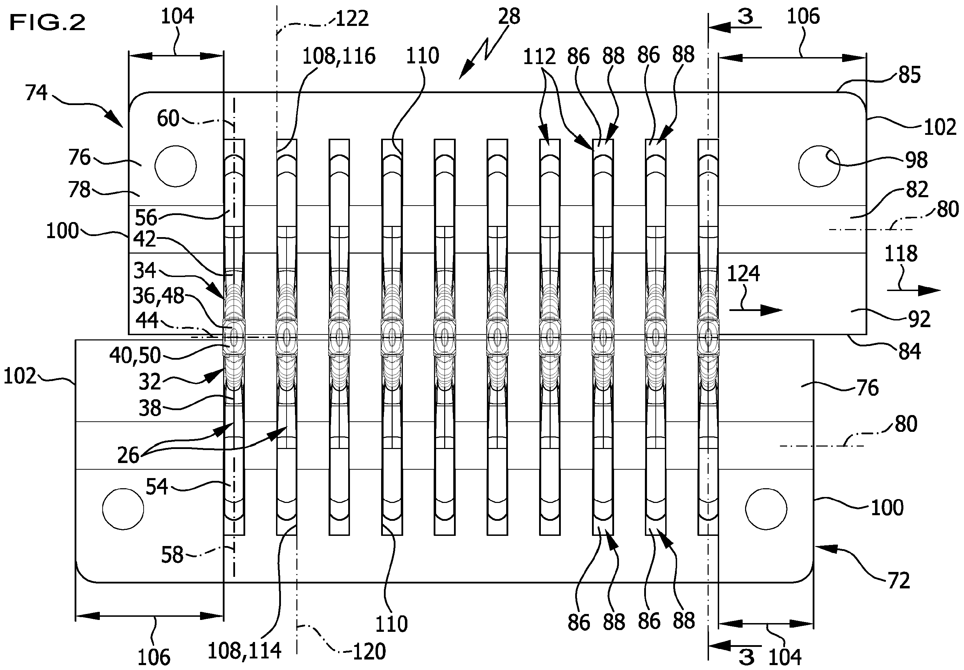

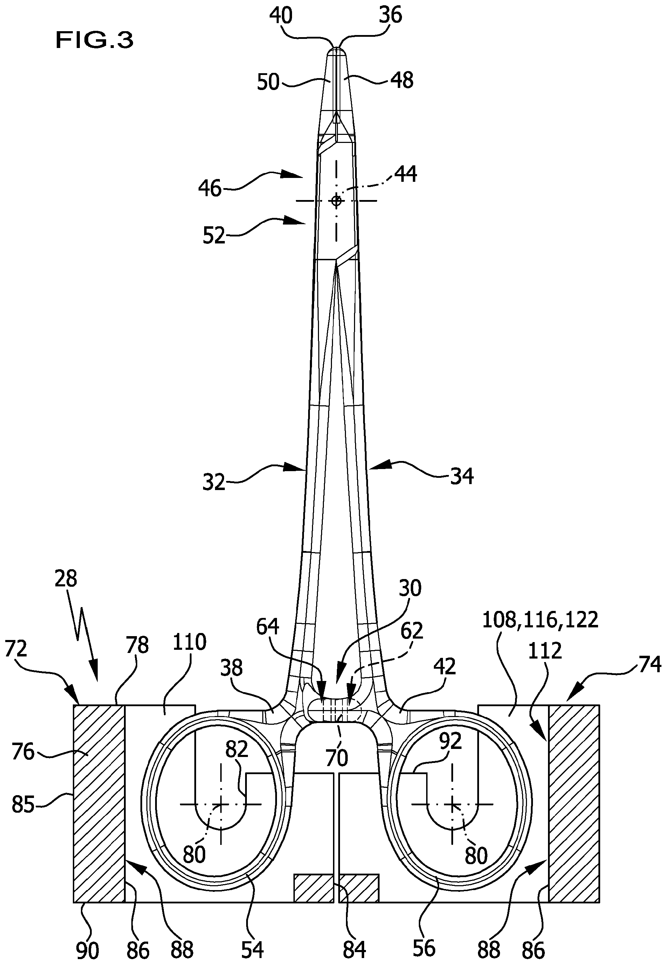

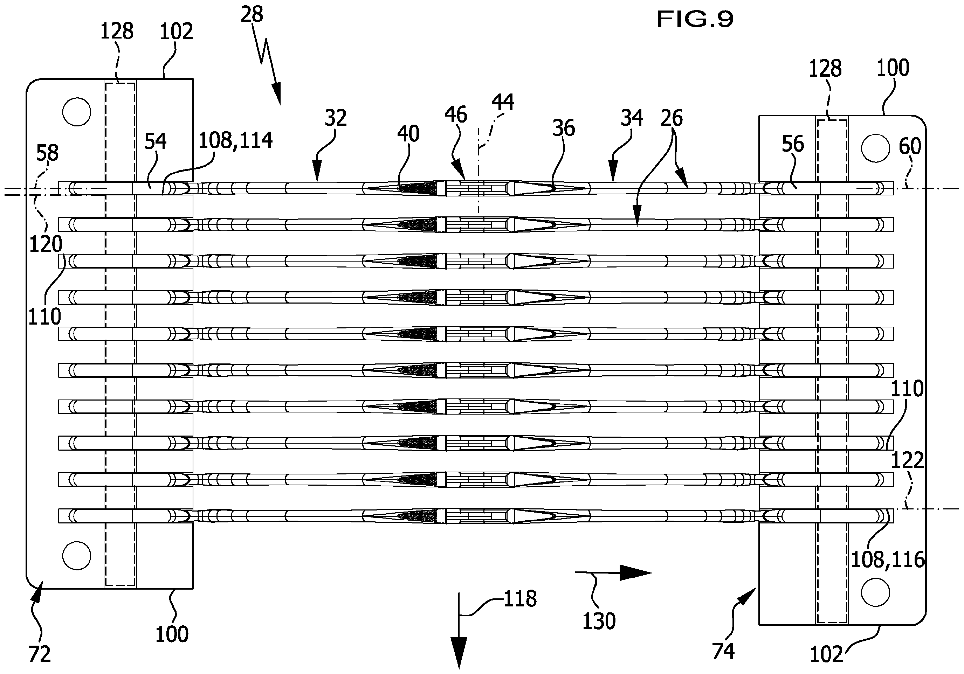

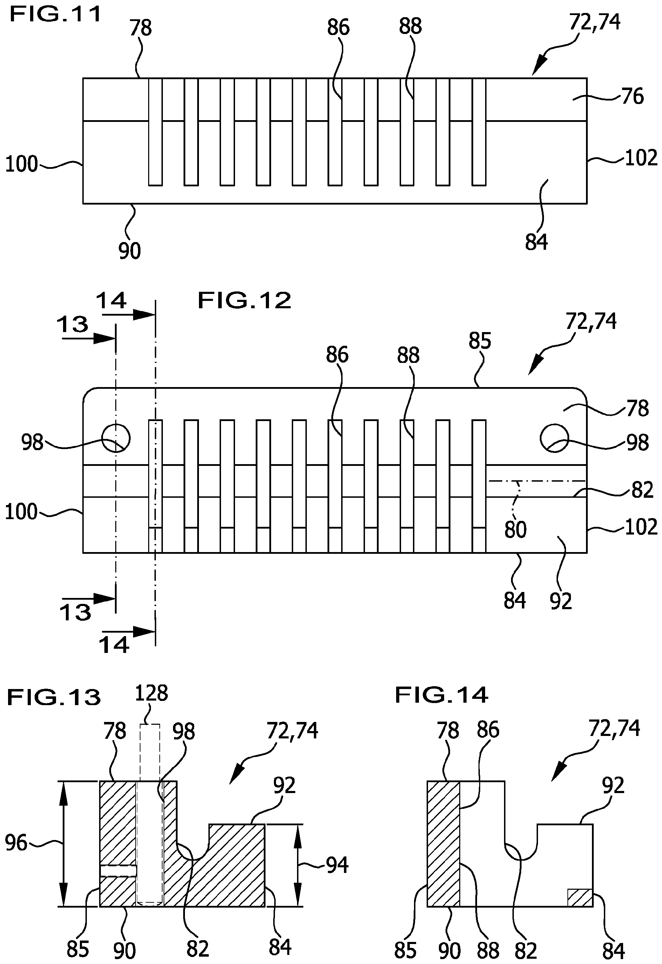

Die Ringinstrumente 26 sind wie schematisch in Figur 1 dargestellt in geordneter Weise im Siebkorb 10 aufgenommen. Hierfür dienen zwei identisch ausgebildete Entsperrvorrichtungen 28, deren Aufbau und Funktionsweise nachfolgend in Verbindung mit den Figuren 2 bis 14 näher erläutert wird.

Das in den Figuren dargestellte Ausführungsbeispiel der Entsperrvorrichtung 28 dient zum Entsperren von zwei oder mehr Ringinstrumenten 26, die Sperreinrichtungen 30 umfassen. Ferner umfassen die Ringinstrumente 26 eine erste Branche 32 und eine zweite Branche 34. Die erste Branche 32 weist ein erstes distales Ende 36 und ein erstes proximales Ende 38 auf. Die zweite Branche 34 weist ein zweites distales Ende 40 und ein zweites proximales Ende 42 auf.

Die beiden Branchen 32 und 34 sind um eine Schwenkachse 44 verschwenkbar aneinander gelagert.

Im Bereich der Schwenkachse 44 ist bei dem in den Figuren 1 bis 8 dargestellten Ringinstrumenten 26 ein Kastenschluss 46 ausgebildet.

Die distalen Enden 36 und 40 sind in Form von Klemmbacken 48 und 50 ausgebildet.

Die Branchen 32 und 34 erstrecken sich stabförmig und im Wesentlichen geradlinig vom durch den Kastenschluss 46 definierten Schlussbereich 52 bis zu den proximalen Enden 38 und 42.

Am ersten proximalen Ende 38 ist ein erster Ring 54 angeordnet. Am zweiten proximalen Ende 42 ist ein zweiter Ring 56 angeordnet.

Der erste Ring 54 definiert eine erste Ringebene 58, der zweite Ring 56 eine zweite Ringebene 60. Die erste Ringebene 58 und die zweite Ringebene 60 des Ringinstruments 26 verlaufen parallel zueinander.

Die Schwenkachse 44 verläuft bei dem in den Figuren dargestellten Ausführungsbeispiel des Ringinstruments 26 quer, nämlich senkrecht, zur ersten Ringebene 58 und zur zweiten Ringebene 60.

Die Sperreinrichtung 30 umfasst zwei identische Sperrelemente 62 und 64 die jeweils nahe den Ringen 54 beziehungsweise 56 von den proximalen Enden 38 beziehungsweise 40 aufeinander zu weisend abstehend angeordnet beziehungsweise ausgebildet sind.

Die Sperrelemente 62 und 64 umfassen jeweils eine Verzahnung 66 beziehungsweise 68. Die Verzahnungen 66 und 68 umfassen jeweils drei hinterschnittene Sperrzähne 70, die jeweils auf die Sperrzähne 70 der jeweils anderen Verzahnung 66 beziehungsweise 68 hin gerichtet sind.

In einer schematisch in den Figuren 1 bis 4 dargestellten Sperrstellung der Ringinstrumente 26 stehen die zusammenwirkenden Sperrelemente 62 und 64 in Eingriff, indem die Sperrzähne 70 der Verzahnungen 66 und 68 ineinandergreifen.

Die Sperrelemente 62 und 64 sind derart ausgebildet und angeordnet, dass die Ringinstrumente 26 beim Schließen derselben, also wenn die Ringe 54 und 56 durch Verschwenken der Branchen 32 und 34 aufeinander zu bewegt werden, ineinandergreifen. Durch die jeweils drei vorgesehen Sperrzähne 70 können die Ringinstrumente 26 in drei unterschiedlich angenäherten Stellungen gegen ein Öffnen, also ein voneinander weg Bewegen sowohl der distalen Enden 36 und 40 als auch der Ringe 54 und 56 jeweils voneinander, gesperrt werden. In der Sperrstellung stehen die Sperrelemente 62 und 64 kraft- und/oder formschlüssig in Eingriff.

Um die Ringinstrumente 26 von der Sperrstellung in eine Entsperrstellung zu überführen, in welcher die Sperrelemente 62 und 64 außer Eingriff stehen, müssen die proximalen Enden 38 und 42 lediglich etwas aufeinander zu und dann die Sperrelemente 62 und 64 etwas voneinander weg in einer Richtung parallel zur Schwenkachse 44 bewegt werden, um die Verzahnungen 66 und 68 außer Eingriff zu bringen. Dieses Entsperren kann ein Anwender beispielsweise von Hand dadurch durchführen, dass er mit seinem Daumen in den ersten Ring 54 greift und mit seinem Zeigefinger in den zweiten Ring 56. So kann er jedes Ringinstrument 26 einzeln von Hand entsperren.

Die Entsperrvorrichtung 28 ermöglicht es einem Anwender dagegen, zwei oder mehr Ringinstrumente 26, bei dem in den Figuren 1 bis 14 dargestellten Ausführungsbeispiel der Entsperrvorrichtung 28 maximal zehn Ringinstrumente 26, in einem Arbeitsgang zu öffnen, und zwar gleichzeitig. Dies wird durch den besonderen Aufbau der Entsperrvorrichtung 28 ermöglicht. Diese umfasst einen Aufnahmekörper 72 und einen Entsperrkörper 74. Bei der Entsperrvorrichtung 28 sind der Aufnahmekörper 72 und der Entsperrkörper 74 identisch ausgebildet.

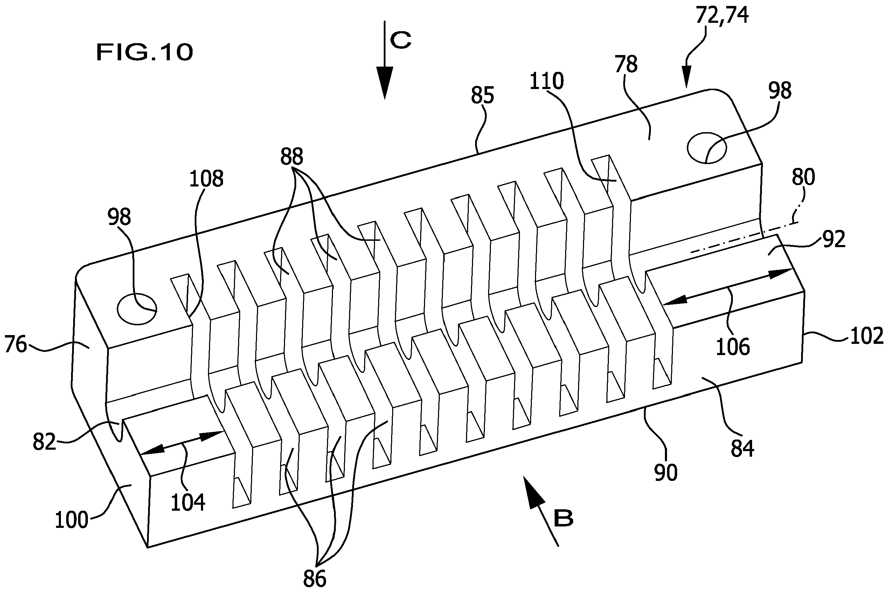

Der Aufbau des Aufnahmekörpers 72 wird nachfolgend in Verbindung mit den Figuren 10 bis 14 näher erläutert.

Der Aufnahmekörper 72 ist aus einem quaderförmigen Grundkörper 76 ausgebildet, an dem ausgehend von der Aufnahmekörperoberseite 78 eine sich parallel zu einer vom Aufnahmekörper 72 definierten Längsrichtung 80 erstreckende nutförmige Längsdurchbrechung 82 ausgebildet ist.

Von einer sich parallel zur Längsrichtung 80 erstreckenden Längsseite 84 sind bei dem dargestellten Ausführungsbeispiel insgesamt zehn Aufnahmeschlitze 86 ausgebildet, die jeweils eine Ringaufnahme 88 zum Aufnehmen eines ersten Rings 54 eines Ringinstruments 26 bilden.

Die Aufnahmeschlitze 86 erstrecken sich von der Aufnahmekörperoberseite 78 bis zu einer Aufnahmekörperunterseite 90 des Aufnahmekörpers 72. Die Aufnahmeschlitze 86 eröffnen den Aufnahmekörper 72 nur auf der Aufnahmekörperoberseite 78, der Längsseite 84 sowie der Aufnahmekörperunterseite 90.

Der Grundkörper 76 ist ausgehend von der Aufnahmekörperoberseite 78 ausgehend von der Längsdurchbrechung 82 zur Längsseite 84 hin etwas zurückgesetzt und definiert eine durch die Aufnahmeschlitze 86 unterbrochene Seitenfläche 92. Ein Abstand 94 zwischen der Seitenfläche 92 und der Aufnahmekörperunterseite 90 ist deutlich kleiner als ein Abstand 96 der Aufnahmekörperoberseite 78 von der Aufnahmekörperunterseite 90. Bei dem in den Figuren dargestellten Ausführungsbeispiel beträgt der Abstand 94 etwa 70% des Abstands 96.

Ferner sind am Aufnahmekörper 72 zwei Führungselementaufnahmen 98 ausgebildet, und zwar in Form von Bohrungen, die den Aufnahmekörper 72 von der Aufnahmekörperoberseite 78 bis zur Aufnahmekörperunterseite 90 durchsetzen und senkrecht zu diesen verlaufen.

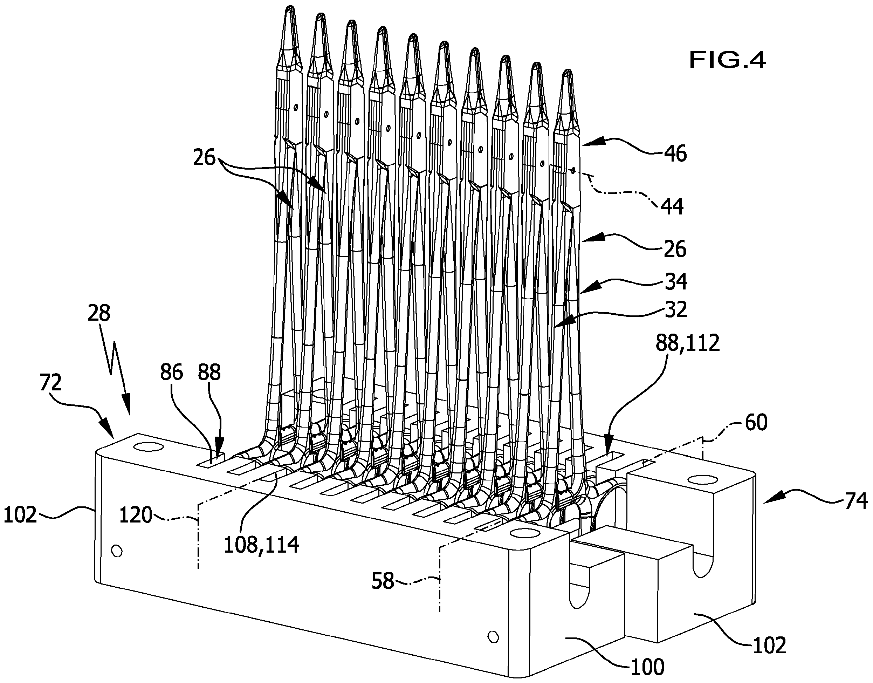

Wie bereits erwähnt ist bei dem Ausführungsbeispiel der Entsperrvorrichtung 28 der Entsperrkörper 74 identisch mit dem Aufnahmekörper 72 ausgebildet. Der Aufnahmekörper 72 und der Entsperrkörper 74 werden, wie in den Figuren 2 und 4 schematisch dargestellt, mit den Längsseiten 84 aneinander angelegt, so dass sich jeweils zwei Ringaufnahmen 88 gegenüberliegen. In die Ringaufnahmen 88 des Aufnahmekörpers 72 werden die ersten Ringe 54 der Ringinstrumente 26 eingesetzt, n die Ringaufnahmen 88 des Entsperrkörpers 74 die zweiten Ringe 56 derselben.

Die Ringaufnahmen 88 sind parallel zu ersten und zweiten Stirnseiten 100 beziehungsweise 102 des Aufnahmekörpers 72 ausgerichtet. Ein Abstand 104 der der ersten Stirnseite 100 nächstgelegenen Ringaufnahme 88 ist kleiner als ein Abstand 106 der der zweiten Stirnseite 102 nächstgelegenen Ringaufnahme 88.

Die Ringaufnahmen 88 sind von zwei parallel zueinander verlaufenden Seitenfläche begrenzt, und zwar von einer ersten Seitenfläche 108, die in Richtung auf die zweite Stirnseite 102 hin weist, und einer zweiten Seitenfläche 110, die in Richtung der ersten Stirnseite 100 weist.

Die Ringaufnahmen 88 des Entsperrkörpers 74 bilden Entsperrelemente 112, die mit den zweiten Ringen 56 der Ringinstrumente 26 zusammenwirken.

Die Ringaufnahmen 88 des Aufnahmekörpers 72 weisen einer Widerlagerfläche 114 für die in der Ringaufnahme 88 aufgenommenen ersten Ringe 54 auf. Die Widerlagerfläche 114 wird gebildet durch die erste Seitenfläche 108 und verläuft parallel zur ersten Ringebene 58.

Die Entsperrelemente 112 weisen jeweils eine Entsperrfläche 116 auf, die durch die erste Seitenfläche 108 einer Ringaufnahme 88 am Entsperrkörper 74 definiert wird. Die Widerlagerflächen 114 definieren jeweils eine Widerlagerebene 120. Die Entsperrflächen 116 definieren eine Entsperrebene 122.

Um die in die Ringaufnahmen 88 am Aufnahmekörper 72 und am Entsperrkörper 74 eingesetzten Ringinstrumente 26 zu entsperren, werden der Aufnahmekörper 72 und der Entsperrkörper 74 in einer Betätigungsrichtung 118 relativ zueinander bewegt. Die Betätigungsrichtung ist in Figur 2 schematisch durch den Pfeil 118 symbolisiert.

Die Relativbewegung des Aufnahmekörpers 72 und des Entsperrkörpers 74 in der Betätigungsrichtung 118 führt dazu, dass sich die zweiten Stirnseiten 102 voneinander entfernen. Bei dieser Bewegung greifen die Entsperrelemente 112 an den zweiten Ringen 56 an und bewegen diese relativ zu den ersten Ringen 54 in einer durch den Pfeil 124 symbolisierten Entsperrrichtung quer, nämlich senkrecht, zur ersten Ringebene 58 und zur zweiten Ringebene 60, um die Sperreinrichtungen 30 der Ringinstrumente 26 von der Sperrstellung in die Entsperrstellung zu überführen.

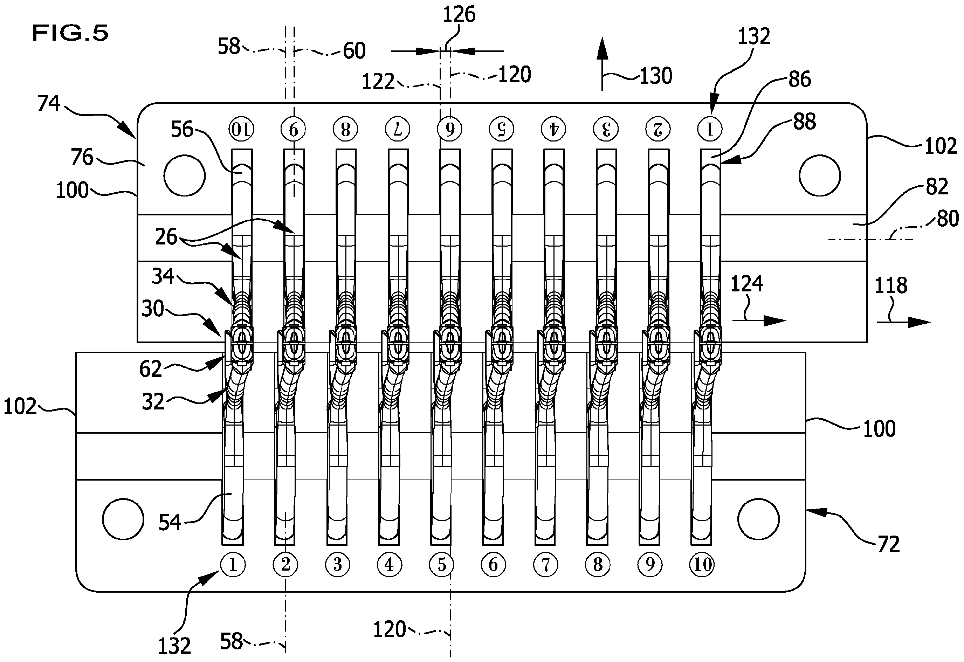

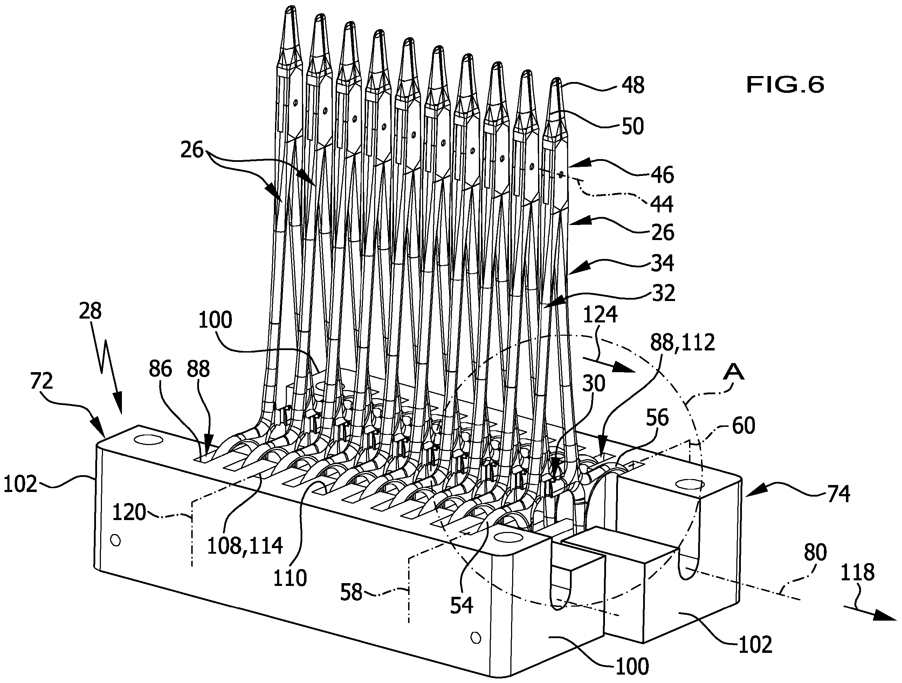

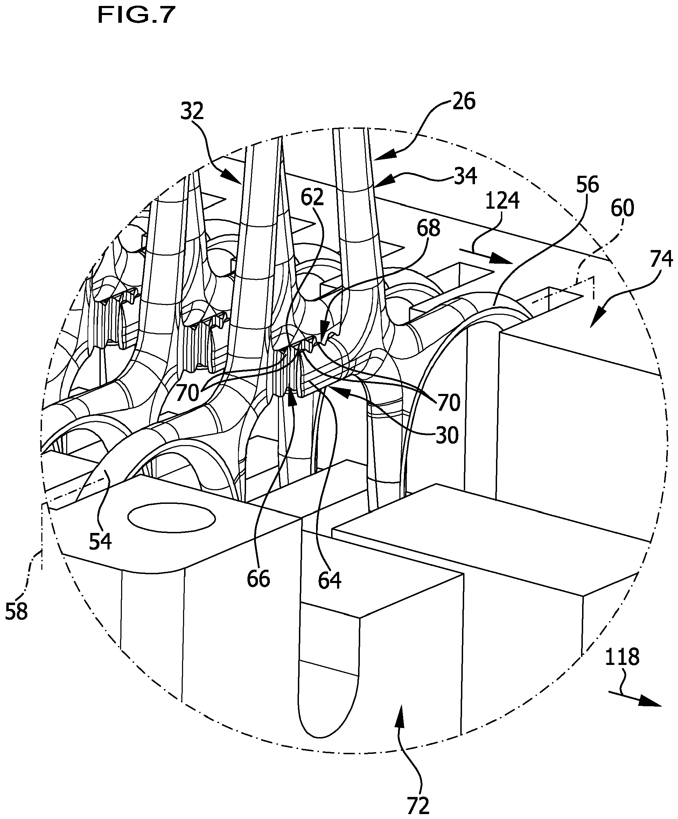

Bei der Relativbewegung des Aufnahmekörpers 72 und des Entsperrkörpers 74 liegen die zweiten Ringe 56 jeweils an einer Entsperrfläche 116 an. Durch die Relativbewegung nimmt ein Abstand 126 zwischen der Widerlagerebene 120 und der Entsperrebene 122 von Ringaufnahmen 88 und Entsperrelementen 112, die einander zugeordnet sind zum Lösen der Sperreinrichtung 30 eines Ringinstruments 26, beim Bewegen des Entsperrkörpers 74 und des Aufnahmekörpers 72 relativ zueinander in der Betätigungsrichtung 118 ab. Dadurch werden die ersten Ringe 54 und die zweiten Ringe 56 der Ringinstrumente 26 etwas in Längsrichtung 80 gegeneinander versetzt, so dass die erste Ringebene 58 und die zweite Ringebene 60, die bei gesperrten Ringinstrumenten 26, die in der Entsperrvorrichtung 28 aufgenommen sind, zusammenfallen, auseinander bewegt werden. Gleichzeitig nimmt der Abstand 126 ab. Diese Effekte sind schematisch in Figur 5 dargestellt. Durch diese seitliche Bewegung der ersten Ringe 54 und der zweiten Ringe 56 gelangen die Sperrelemente 62 und 64 der Sperreinrichtungen 30 außer Eingriff, wie dies schematisch in den Figuren 6 und 7 dargestellt ist.

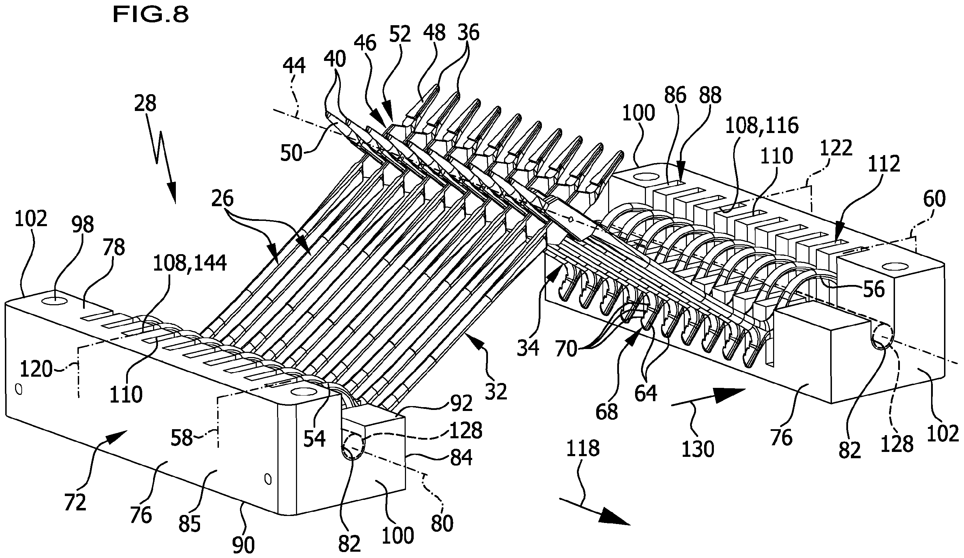

Die Entsperrvorrichtung 28 kann optional zwei stabförmige Halteglieder 128 umfassen, die in einer Haltestellung, wie sie schematisch in Figur 8 dargestellt ist, in den Längsdurchbrechungen 82 des Aufnahmekörpers 72 und des Entsperrkörpers 74 aufgenommen sind. Da die Längsdurchbrechung 82 die Ringaufnahmen 88 durchsetzt, durchgreifen die Halteglieder 128 einerseits alle ersten Ringe 54 und andererseits alle zweiten Ringe 56 der Ringinstrumente 26.

Sind die Halteglieder 128 in den Längsdurchbrechungen 82 aufgenommen, können der Aufnahmekörper 72 und der Entsperrkörper 74 zum Öffnen der Ringinstrumente 26 nach dem beschriebenen Überführen von der Sperrstellung in die Entsperrstellung durch Verschwenken der ersten und zweiten Ringe 54 und 56 voneinander weg um die Schwenkachse 44 in einer Öffnungsrichtung, die in Figur 8 durch den Pfeil 130 symbolisiert ist, voneinander weg bewegt werden. Die Öffnungsrichtung 130 verläuft quer, nämlich senkrecht, zur Betätigungsrichtung 118.

Wie beschrieben verläuft die Betätigungseinrichtung 118 parallel zur Längsrichtung 80. Bei dem beschriebenen Ausführungsbeispiel der Entsperrvorrichtung 28 sind der Aufnahmekörper 72 und der Entsperrkörper 74 in einer Richtung quer, nämlich senkrecht, also in der Betätigungseinrichtung 118, zu den Ringaufnahmen 88 bewegbar. Damit ist die Betätigungsrichtung 118 quer zu den ersten Ringebenen 58 der Ringinstrumente 26 verlaufend.

Bei dem beschriebenen Ausführungsbeispiel der Entsperrvorrichtung 28 entspricht eine Anzahl der Ringaufnahmen 88 des Aufnahmekörpers 72 einer Anzahl der Entsperrelemente 112 des Entsperrkörpers 74.

Ferner sind die Ringaufnahmen 88 des Aufnahmekörpers 72 und die Entsperrelemente 112 fortlaufend nummeriert. Eine solche Nummerierung 132 ist schematisch in Figur 5 auf der jeweiligen Aufnahmekörperoberseite 80 angebracht, beispielsweise durch Bedrucken, Laserbeschriften oder Prägen.

Wie bereits erwähnt können mit der Entsperrvorrichtung 78 aufgrund ihrer Ausgestaltung die Sperreinrichtungen 30 der Ringinstrumente 26 gleichzeitig außer Eingriff gebracht werden zum Entsperren der Ringinstrumente 26.

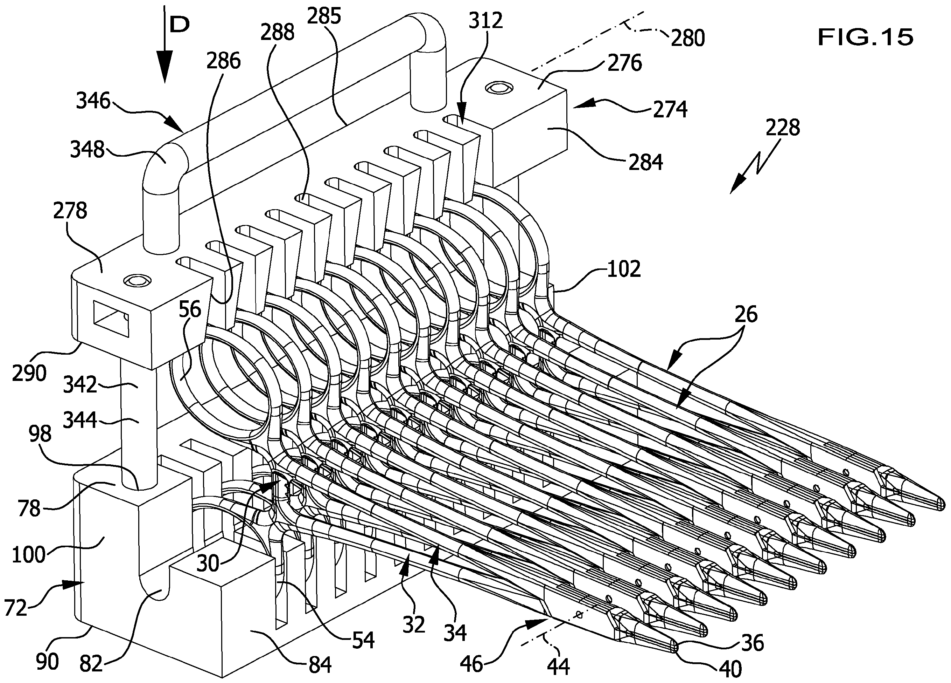

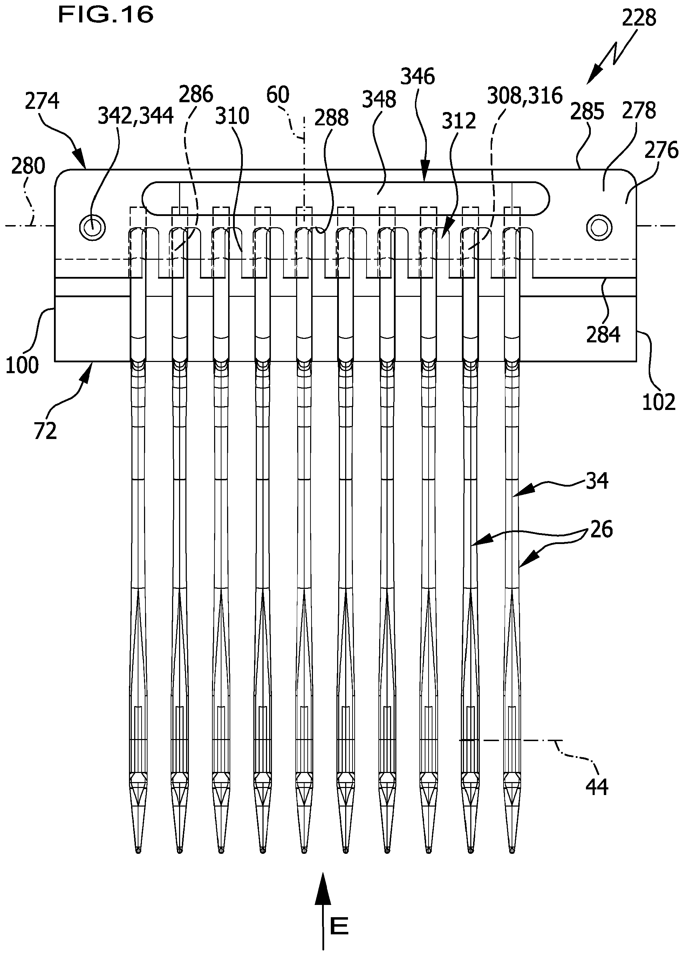

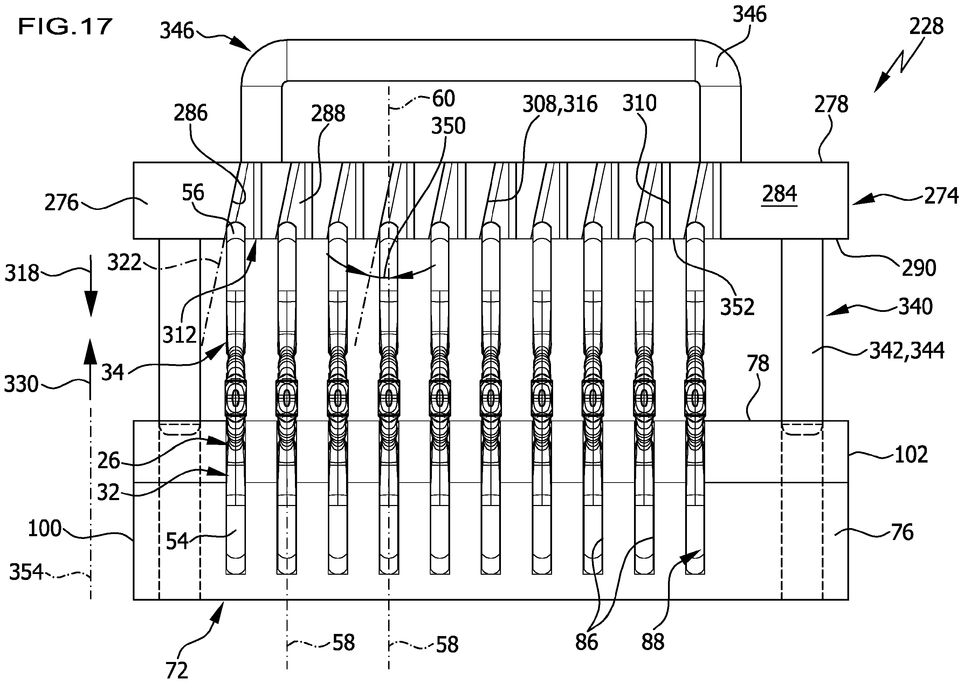

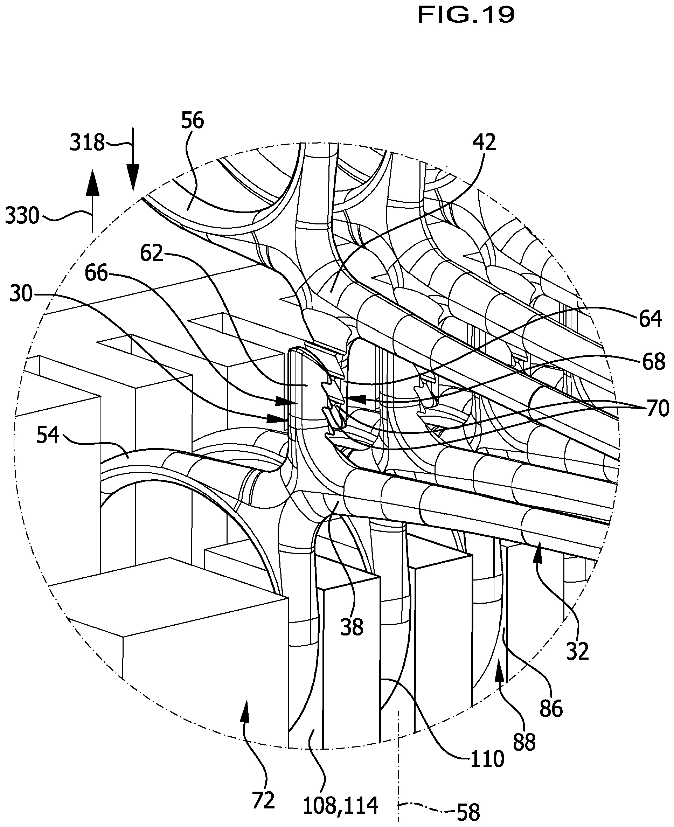

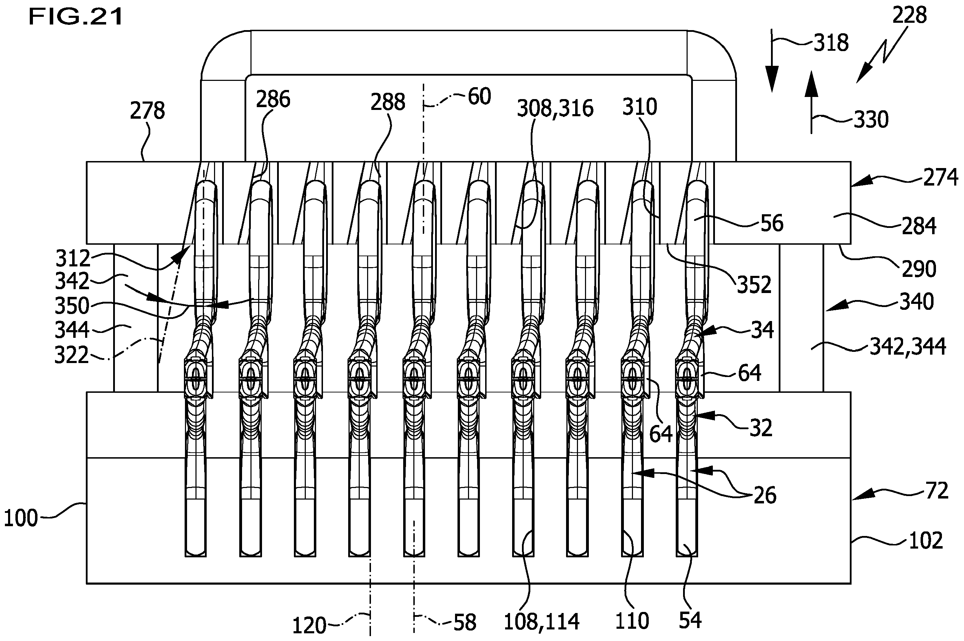

In den Figuren 15 bis 22 ist schematisch ein zweites Ausführungsbeispiel einer Entsperrvorrichtung dargestellt und insgesamt mit dem Bezugszeichen 228 bezeichnet. Auch diese Entsperrvorrichtung 228 ist ausgebildet zum Entsperren mehrerer Ringinstrumente 26, deren Aufbau oben bereits eingehend beschrieben wurde.

Die Entsperrvorrichtung 228 umfasst einen Aufnahmekörper 72 und einen Entsperrkörper 274. Der Aufnahmekörper 72 ist identisch ausgebildet mit dem Aufnahmekörper 72 der Entsperrvorrichtung 28. Auf obige Beschreibung des Aufbaus des Aufnahmekörpers 72 wird daher zur Vermeidung von Wiederholungen verwiesen.

Der Entsperrkörper 274 unterscheidet sich vom Aufnahmekörper 72 in seinem Aufbau. Der Entsperrkörper 274 ist aus einem quaderförmigen Grundkörper 276 ausgebildet. Der Grundkörper 276 weist eine Entsperrkörperoberseite 278 oder eine Entsperrkörperunterseite 290 auf. Ferner definiert der Grundkörper 276 eine Längsrichtung 280 und weist zwei voneinander weg weisende Längsseiten 284 und 286 auf.

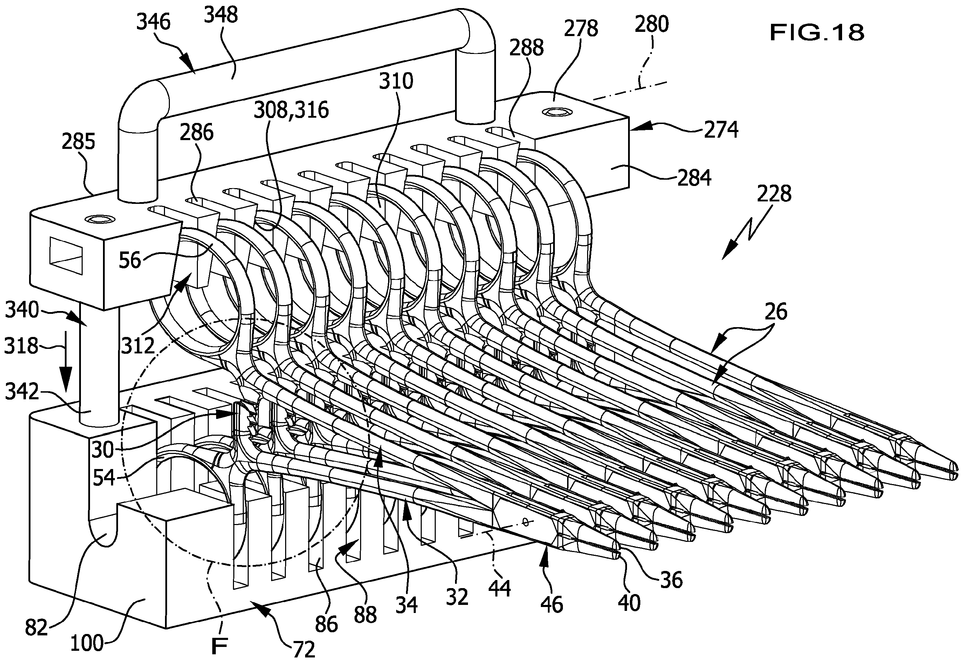

Die Entsperrvorrichtung228 umfasst ferner eine Linearbewegungsführungseinrichtung 340, mittels derer der Aufnahmekörper 72 und der Entsperrkörper 274 zum Führen einer Bewegung relativ zueinander gekoppelt sind. Die Linearbewegungsführungseinrichtung 340 umfasst zwei geradlinig ausgebildete Führungselemente 342 in Form von Führungsstäben 344. Die Führungsstäbe 344 sind fest mit dem Entsperrkörper 274 verbunden und stehen senkrecht von der Entsperrkörperunterseite 290 vor. Die Führungsstäbe 344 greifen in die Führungselementaufnahmen 98 am Aufnahmekörper 72 ein. Dies ermöglicht eine Führung einer Bewegung des Entsperrkörpers 274 in einer Betätigungsrichtung, die in Figur 18 schematisch durch den Pfeil 318 symbolisiert ist, in Richtung auf den Aufnahmekörper 72 hin.

Auf der Entsperrkörperoberseite 278 ist am Entsperrkörper 274 ein Griffelement 346 in Form eines U-förmigen Haltebügels 348 angeordnet. Parallele zu einander verlaufende Schenkel des Haltebügels 348 erstrecken sich parallel zu den Führungselementen 342.

Der Entsperrkörper 274 weist eine Mehrzahl von Entsperrelementen 312 auf. Diese sind in Form Ringaufnahmen 288 ausgebildet. Die Ringaufnahmen 288 werden definiert durch Aufnahmeschlitze 286, die im Entsperrkörper 274 ausgebildet sind, und zwar ausgehend von der Längsseite 284. Die Aufnahmeschlitze 286 erstrecken sich von der Entsperrkörperoberseite 278 bis zur Entsperrkörperunterseite 290.

Die Aufnahmeschlitze 286 werden seitlich begrenzt von einer ersten Seitenfläche 308 und einer zweiten Seitenfläche 310. Die erste Seitenfläche 308 weist in Richtung der ersten Stirnseite 100, die zweite Seitenfläche 310 in Richtung der zweiten Stirnseite 102. Die zweite Seitenfläche 310 verläuft parallel zu den Seitenflächen 108 und 110 des Aufnahmekörpers 72. Die erste Seitenfläche 308 definiert eine Entsperrfläche 316, die eine Entsperrebene 322 definiert. Die Entsperrflächen 316 definieren also Entsperrebenen 322, die relativ zu den zugeordneten Widerlagerebenen 120, die von den Widerlagerflächen 114 des Aufnahmekörpers 72 definiert werden, um einen Neigungswinkel 350 geneigt sind, so dass sich die Ringaufnahme 288 am Entsperrkörper 274 vom Aufnahmekörper 72 weg verjüngt.

Der Neigungswinkel 350 liegt bei dem in den Figuren dargestellten Ausführungsbeispiel der Entsperrvorrichtung 228 in einem Bereich von etwa 10° bis etwa 50°. Die Neigungswinkel 350 aller Entsperrflächen 316 sind identisch.

Ferner ist ein Abstand von in Richtung auf den Aufnahmekörper 72 hin weisenden Enden 352 der Entsperrflächen 316 von der Entsperrkörperunterseite 290, die in Richtung auf den Aufnahmekörper 72 hin weist, identisch. Die Entsperrflächen 316 erstrecken sich von der Entsperrkörperoberseite 278 bis zur Entsperrkörperunterseite 290.

Die Funktionsweise der Entsperrvorrichtung 228 wird nachfolgend näher erläutert.

Der Aufnahmekörper 72 und der Entsperrkörper 274 sind in der Betätigungsrichtung 318 parallel zu den Ringaufnahmen 88 und damit parallel zu den ersten Ringebenen 58 der Ringinstrumente 26 bewegbar zum Überführen der Sperreinrichtungen 30 der Ringinstrumente 26 von der Sperrstellung in die Entsperrstellung. Die Betätigungsrichtung 318 verläuft somit in einer Betätigungsebene 354, die sich parallel zu den ersten und zweiten Ringebenen 58 und 60 erstreckt.

Der Aufnahmekörper 72 und der Entsperrkörper 274 sind relativ zueinander in der Betätigungsebene 354 verschiebbar. Eine solche Verschiebebewegung wird mittels der Linearbewegungsführungseinrichtung 340 geführt. Die Betätigungsrichtung 318 verläuft quer, nämlich senkrecht, zur Längsrichtung 80 des Aufnahmekörpers 72.

Bei einer Bewegung des Entsperrkörpers 274 in der Betätigungsrichtung 318 auf den Aufnahmekörper 72 hin liegen die zweiten Ringe 56 jeweils an einer der Entsperrflächen 316 des Entsperrkörpers 274 an und gleiten mit fortschreitender Bewegung des Entsperrkörpers 274 auf den Aufnahmekörper 72 hin an den Entsperrflächen 316 auf. Beim Aufgleiten werden die zweiten Ringe 56 aus der ersten Ringebene 58 herausbewegt in Richtung auf die erste Stirnseite 100 hin. Durch diese Seitwärtsbewegung der zweiten Ringe 56 werden die in der Sperrstellung ineinandergreifenden Sperrelemente 62 und 64 der Sperreinrichtung 30 außer Eingriff gebracht.

In den Figuren 15, 16 und 17 ist die Entsperrvorrichtung 228 schematisch dargestellt mit zehn aufgenommenen Ringinstrumenten 26, die die Sperrstellung einnehmen.

In den Figuren 18, 19 und 21 ist schematisch dargestellt, wie die Sperreinrichtungen 30 entsperrt werden durch außer Eingriff Bringen der Sperrelemente 62 und 64.

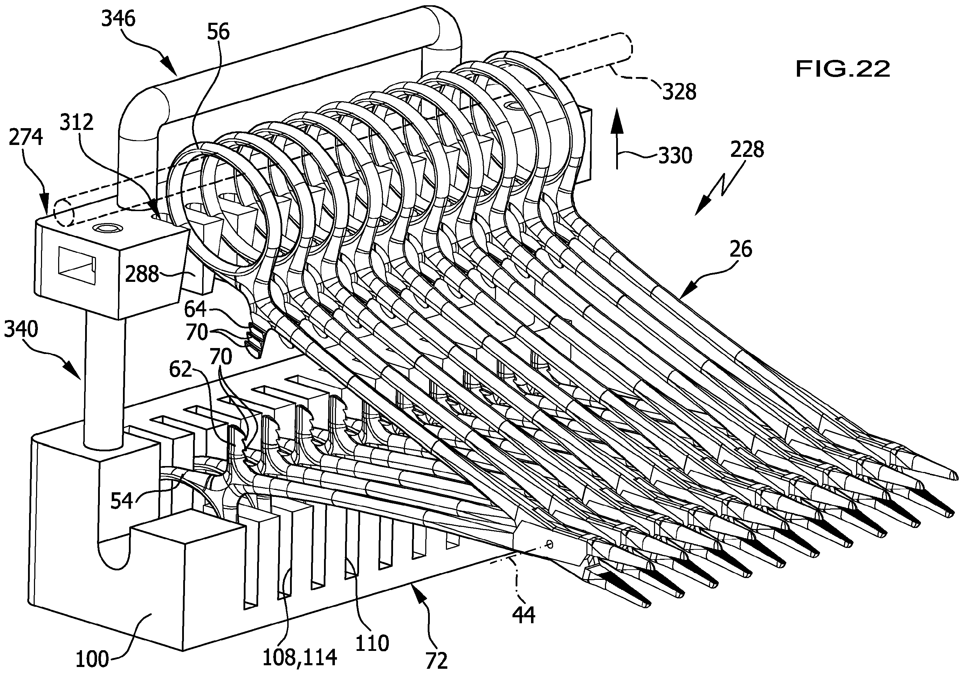

Wird der Entsperrkörper 274 noch weiter in Richtung auf den Aufnahmekörper 72 hin bewegt, rutschen die zweiten Ringe 56 auf der Entsperrkörperoberseite 278 aus den Ringaufnahmen 288 heraus. Dabei federn die zweiten Ringe 56 wieder in ihre Grundstellung zurück, in der die erste Ringebene 58 und die zweite Ringebene 60 jedes Ringinstruments 26 zusammenfallen. Nun kann beispielsweise ein stabförmiges Halteglied 328 durch die zweiten Ringe 56 hindurchgeführt werden. Dies ist schematisch in Figur 22 dargestellt.

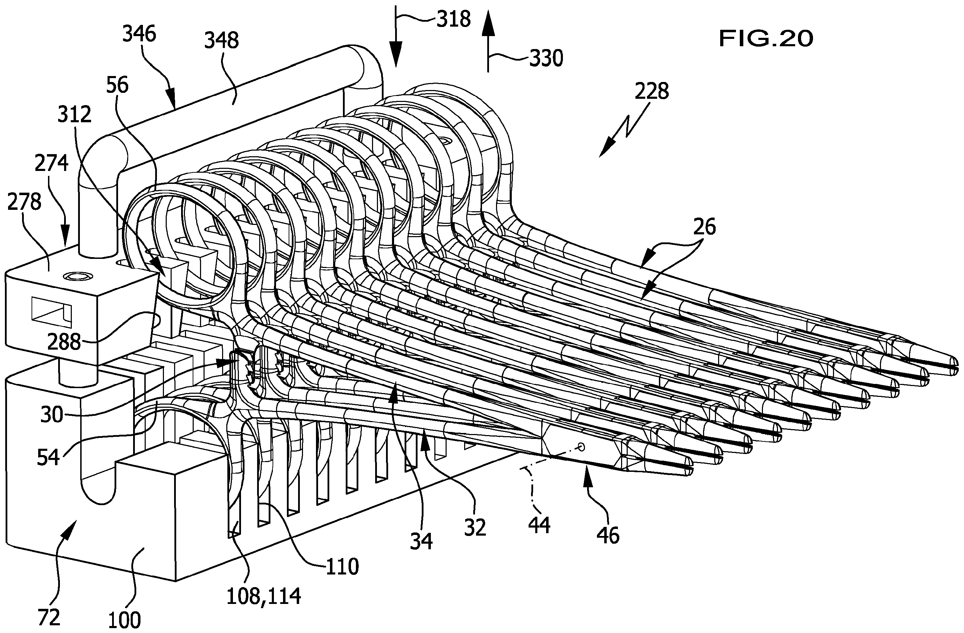

Wird der Entsperrkörper 274 aus der in Figur 20 dargestellten angenäherten Stellung wieder in die Ausgangsstellung zurückbewegt, in der der Entsperrkörper 274 vom Aufnahmekörper 72 maximal weit beabstandet ist, lassen sich die Ringinstrumente 26 öffnen. Die Bewegung zum Öffnen erfolgt in einer durch den Pfeil 330 in Figur 22 symbolisierten Öffnungsrichtung. Beim Öffnen der Ringinstrumente 26 werden die Ringe 54 und 56 relativ zueinander um die Schwenkachse 44 verschwenkt. In dieser in Figur 22 dargestellten geöffneten Stellung kann die Entsperrvorrichtung 228 zur Lagerung der Ringinstrumente 26 im Siebkorb 10 dienen, und zwar sowohl beim Waschen als auch beim Heißdampfsterilisieren der Ringinstrumente 26.

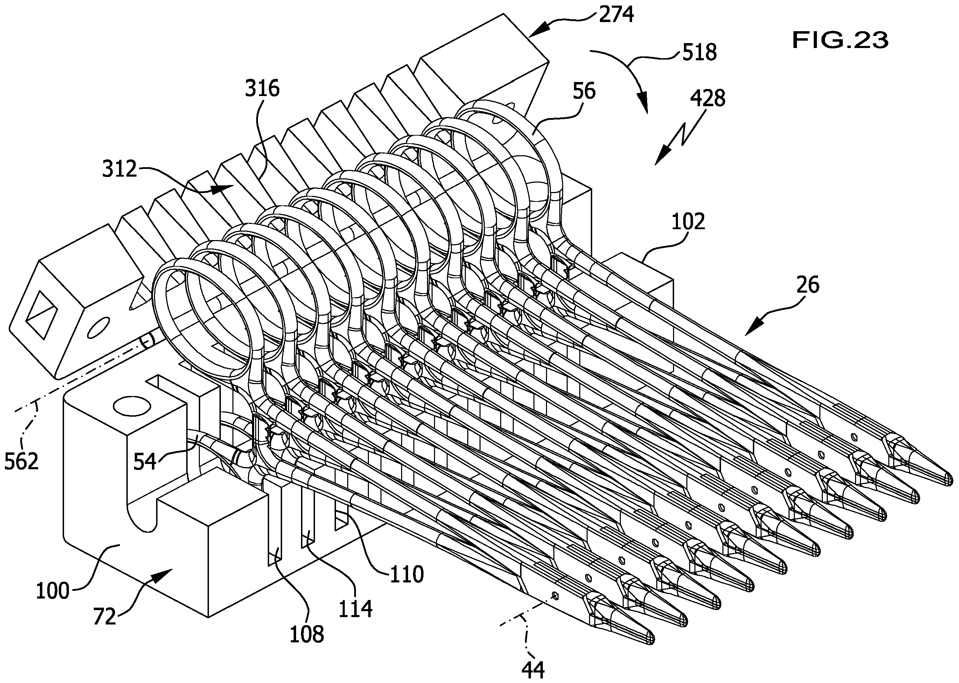

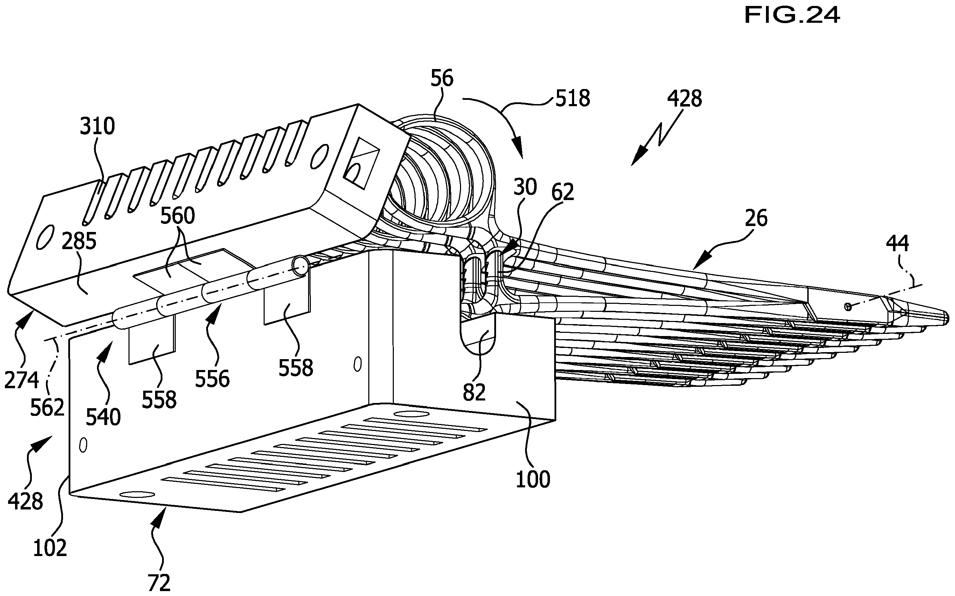

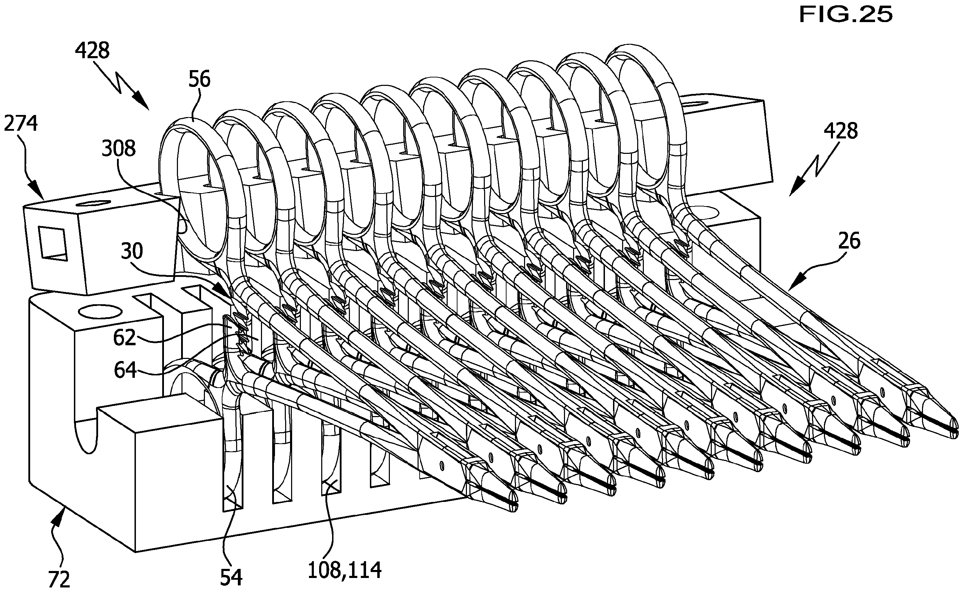

In den Figuren 23 bis 25 ist ein weiteres Ausführungsbeispiel einer insgesamt mit dem Bezugszeichen 428 bezeichneten Entsperrvorrichtung schematisch dargestellt.

Die Entsperrvorrichtung 428 unterscheidet sich von der Entsperrvorrichtung 228 dadurch, dass sie statt der Linearbewegungsführungseinrichtung 340 eine Schwenkbewegungsführungseinrichtung 540 umfasst. Die Schwenkbewegungsführungseinrichtung 540 umfasst ein Schwenklager 556, welches in Form eines Scharniers ausgebildet ist, dessen Bänder 558 und 560 einerseits am Aufnahmekörper 72 und andererseits am Entsperrkörper 274 angeordnet sind. Die Bänder 560 sind an der Längsseite 285 des Entsperrkörpers 274 befestigt, die Bänder 558 an einer von der Längsseite 84 entgegengesetzt weg weisenden Längsseite 85 des Aufnahmekörpers 72. Eine durch das Schwenklager 556 definierte Schwenkachse 562 verläuft somit parallel zu den Längsseiten 85 und 285.

Bei der Entsperrvorrichtung 428 verläuft eine Betätigungsrichtung 518 in einer Betätigungsebene, die senkrecht zur Schwenkachse 562 verläuft, sich also parallel zu den Ringebenen 58 und 60 erstreckt.

Die Funktionsweise der Entsperrvorrichtung 428 entspricht im Wesentlichen dem Funktionsprinzip der Entsperrvorrichtung 228. Der Unterschied der beiden Entsperrvorrichtungen 228 und 428 besteht insbesondere darin, dass bei der Entsperrvorrichtung 428 der Entsperrkörper 274 um die Schwenkachse 562 auf den Aufnahmekörper 72 hin verschwenkt wird, bei der Entsperrvorrichtung 228 wird der Entsperrkörper 274 in Richtung auf den Aufnahmekörper 72 hin verschoben. In beiden Fällen gleiten bei der Bewegung des Entsperrkörpers 274 relativ zum Aufnahmekörper 72 die zweiten Ringe 56 an den Entsperrflächen 316 auf, wodurch die zweiten Ringe 56 seitlich parallel zur Längsrichtung 80 ausgelenkt werden, so dass die Sperrelemente 62 und 64 der Sperreinrichtungen 30 außer Eingriff gelangen können.

Bei der Entsperrvorrichtung 428 kann optional ein Betätigungshebel vorgesehen werden, welcher am Entsperrkörper 274 festgelegt werden kann. Beispielsweise kann dieser Betätigungshebel in Form eines Exzenterhebels ausgebildet werden, um es einem Anwender mit möglichst geringem Kraftaufwand zu ermöglichen, den Entsperrkörper 274 um die Schwenkachse 562 auf den Aufnahmekörper 72 hin zu verschwenken, um alle zehn in der Entsperrvorrichtung 428 aufgenommenen Ringinstrumente 26 gleichzeitig zu Entsperren.

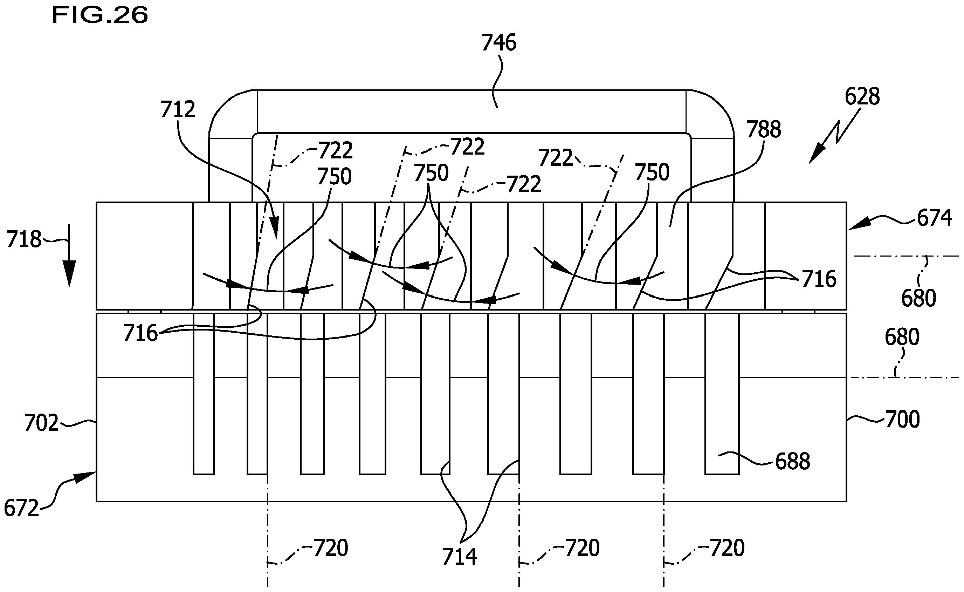

In Figur 26 ist schematisch ein weiteres Ausführungsbeispiel einer Entsperrvorrichtung dargestellt und mit dem Bezugszeichen 628 bezeichnet. Die Entsperrvorrichtung 628 umfasst einen Aufnahmekörper 672 und einen Entsperrkörper 674.

Der Aufnahmekörper 672 ist in seinem Aufbau dem Aufnahmekörper 72 ähnlich. Allerdings nimmt eine Breite der Ringaufnahmen 688 ausgehend von der der zweiten Stirnseite 702 nächstgelegenen Ringaufnahme bis zu der der ersten Stirnseite 700 nächstgelegenen Ringaufnahme 688 zu. Dies ist erforderlich aufgrund der besonderen Ausgestaltung der Entsperrelemente 712.

Die Ringaufnahmen 788 des Entsperrkörpers 674 unterscheiden sich aufgrund einer unterschiedlichen Neigung der Entsperrflächen 716 voneinander. Die von den Entsperrflächen 716 definierten Entsperrebenen 722 schließen mit den von den Widerlagerflächen 714 definierten Widerlagerebenen 720 jeweils einen Neigungswinkel 750 ein, der ausgehend von dem der zweiten Stirnseite 702 nächstgelegenen Entsperrelement 712 bis zu dem der ersten Stirnseite 700 nächstgelegenen Entsperrelement 712 sukzessive zunimmt.

Der Entsperrkörper 674 kann wahlweise relativ zum Aufnahmekörper 672 linear bewegt oder, ähnlich wie bei der Entsperrvorrichtung 428, verschwenkt werden.

Die Entsperrflächen 716 bilden Aufgleitflächen für die zweiten Ringe 56, die in Figur 26 der Übersichtlichkeit wegen nicht dargestellt sind. Aufgrund der unterschiedlichen Neigungswinkel 750 werden nicht alle Sperreinrichtungen 30 der in den Ringaufnahmen 688 und 788 aufgenommenen Ringinstrumente 26 gleichzeitig entsperrt, sondern nacheinander, also sukzessive. Zunächst wird das Ringinstrument 26 entsperrt, dessen zweiter Ring 56 an der der ersten Stirnseite 700 nächstgelegenen Entsperrfläche 716 anliegt. Hier ist der Neigungswinkel 750 am größten, so dass bereits bei einem geringen Vorschub des Entsperrkörpers 674 auf den Aufnahmekörper 672 hin der zweite Ring 56 soweit seitlich ausgelenkt ist, dass die Sperrelemente 62 und 64 der Sperreinrichtung 30 dieses Ringinstruments 26 außer Eingriff gelangen, die anderen Ringinstrumente 26, die in der Entsperrvorrichtung 628 aufgenommen sind, noch die Sperrstellung einnehmen.

Bei der Entsperrvorrichtung 628 nehmen die Neigungswinkel 780 benachbarter Entsperrflächen 716 sukzessive zu. Ein Änderungswinkel, also derjenige Winkel, um den sich die Neigungswinkel 750 benachbarter Entsperrflächen 716 unterscheiden, liegt in einem Bereich von etwa 1° bis etwa 10°.

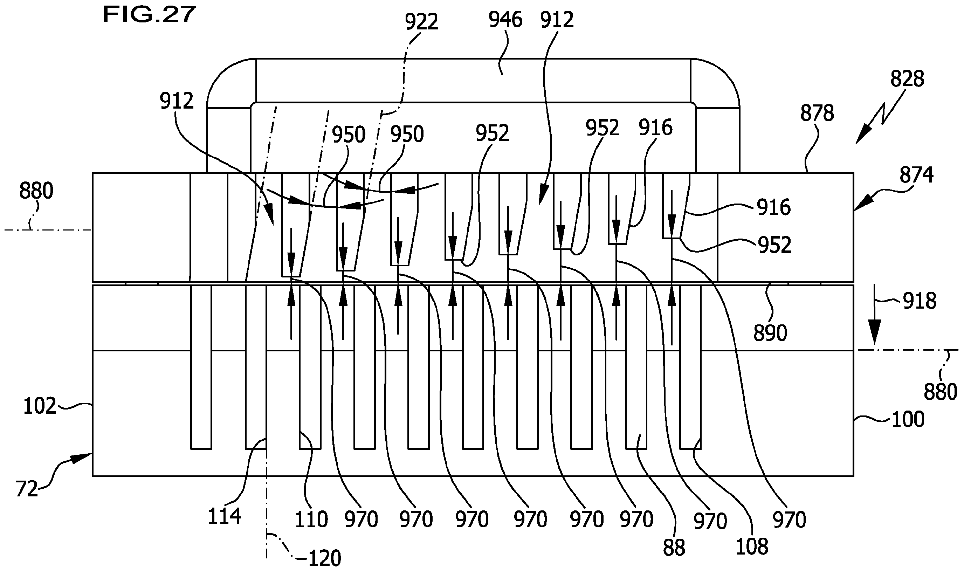

Ein weiteres Ausführungsbeispiel einer insgesamt mit dem Bezugszeichen 828 bezeichneten Entsperrvorrichtung ist schematisch in Figur 27 dargestellt.

Die Entsperrvorrichtung 828 umfasst einen Aufnahmekörper 72, wie er oben bereits eingehend beschrieben wurde.

Der Entsperrkörper 874 ist dem Entsperrkörper 274 ähnlich. Die Entsperrelemente 912 umfassen Entsperrflächen 916, die Entsperrebenen 922 definieren, welche mit den Widerlagerebenen 120 einen Neigungswinkel 950 einschließen. Der Neigungswinkel 950 ist bei allen Entsperrelementen 912 identisch.

Die Entsperrelemente 912 unterscheiden sich von den Entsperrelementen 312 jedoch dadurch, dass ein Abstand 970 von in Richtung auf den Aufnahmekörper 72 hin weisenden Enden 952 der Entsperrflächen 916 von der Entsperrkörperunterseite 890, die in Richtung auf den Aufnahmekörper 72 hin weist, unterschiedlich ist. Bei dem in Figur 27 dargestellten Ausführungsbeispiel nimmt der Abstand 970 ausgehend von dem der zweiten Stirnseite 102 nächstgelegenen Entsperrelement 912 sukzessive von Entsperrelement 912 zu Entsperrelement 912 zu, und zwar bis zu dem der ersten Stirnseite 100 nächstgelegenen Entsperrelement 912.

Zum Entsperren mehrerer Ringinstrumente 26 werden die ersten Ringe 54 in die Ringaufnahmen 88 des Aufnahmekörpers 72 eingesetzt. Wird der Entsperrkörper 874 in der Betätigungsrichtung 918 in Richtung auf den Aufnahmekörper 72 hin verschoben oder auf diesen hin verschwenkt, gelangen die zweiten Ringe 56 der Ringinstrumente 26 sukzessive in Kontakt mit den Entsperrflächen 916, und zwar zunächst der zweite Ring 56 des der zweiten Stirnseite 102 nächstliegend angeordneten Ringinstruments 26 mit der Entsperrfläche 916 des der zweiten Stirnseite 102 nächstgelegenen Entsperrelements 912. So werden mit der Entsperrvorrichtung 828 die Ringinstrumente 26 sukzessive entsperrt, ähnlich wie bei der Entsperrvorrichtung 628, wenngleich bei Entsperrvorrichtung 828 ein anderes Prinzip genutzt wird als bei der Entsperrvorrichtung 628.

In den Figuren nicht dargestellt ist eine optional an den Aufnahmekörpern 72 und 672 angeordnete oder ausgebildete Kopplungseinrichtung zum kraft- und/oder formschlüssigen Koppeln mit dem Siebkorb 10 in einer Kopplungsstellung. Die Kopplungseinrichtung kann insbesondere Kopplungselemente umfassen, die mit den Perforationen 24 des Siebkorbs 10 kraft- und/oder formschlüssig in der Kopplungsstellung in Eingriff stehen. Beispielsweise kann die Kopplungseinrichtung Kopplungsvorsprünge umfassen aus einem Kunststoff, der elastisch ist und in einer Perforation 24 am Siebkorb 10 in der Kopplungsstellung klemmend gehalten ist. Die Kopplungseinrichtung kann ferner auch Haken umfassen, die in die Perforationen 24 eingreifen.

Die oben beschriebenen Entsperrvorrichtungen 28, 228, 428, 628 und 828 sind aus Werkstoffen ausgebildet, die heißdampfsterilisierbar sind. Die beschriebenen Aufnahme- und Entsperrkörper sind aus einem Kunststoff ausgebildet. Dieser kann bei bestimmten Ausführungsbeispielen Polyetheretherketon (PEEK) sein. Die Griffelemente sowie die Führungselemente der Linearbewegungsführungseinrichtung sowie der Schwenkbewegungsführungseinrichtung können aus einem metallischen Werkstoff ausgebildet sein, beispielsweise aus einem Instrumentenstahl.

Mit den beschriebenen Entsperrvorrichtungen 28, 228, 428, 628 und 828 lassen sich zwei oder mehr Ringinstrumente 26 gleichzeitig oder sukzessive auf einfache und für einen Nutzer kraftschonende Weise entsperren sowie bei Bedarf auch in einem Siebkorb lagern, insbesondere zur Aufbereitung der Ringinstrumente 26 in einer Waschmaschine oder einem Heißdampfsterilisationsgerät.

### Bezugszeichenliste

- 10: Siebkorb
- 12: Aufnahmeraum
- 14: Siebkorbboden
- 16: Siebkorbwand
- 18: Siebkorbwand
- 20: Siebkorbwand
- 22: Siebkorbwand
- 24: Perforation
- 26: Ringinstrument
- 28: Entsperrvorrichtung
- 30: Sperreinrichtung
- 32: erste Branche
- 34: zweite Branche
- 36: erstes distales Ende
- 38: erstes proximales Ende
- 40: zweites distales Ende
- 42: zweites proximales Ende
- 44: Schwenkachse
- 46: Kastenschluss
- 48: Klemmbacken
- 50: Klemmbacken
- 52: Schlussbereich
- 54: erster Ring
- 56: zweiter Ring
- 58: erste Ringebene
- 60: zweite Ringebene
- 62: Sperrelement
- 64: Sperrelement
- 66: Verzahnung
- 68: Verzahnung
- 70: Sperrzahn
- 72: Aufnahmekörper
- 74: Entsperrkörper
- 76: Grundkörper
- 78: Aufnahmekörperoberseite
- 80: Längsrichtung
- 82: Längsdurchbrechung
- 84: Längsseite
- 85: Längsseite
- 86: Aufnahmeschlitz
- 88: Ringaufnahme
- 90: Aufnahmekörperunterseite
- 92: Seitenfläche
- 94: Abstand
- 96: Abstand
- 98: Führungselementaufnahme
- 100: erste Stirnseite
- 102: zweite Stirnseite
- 104: Abstand
- 106: Abstand
- 108: erste Seitenfläche
- 110: zweite Seitenfläche
- 112: Entsperrelement
- 114: Wiederlagerfläche
- 116: Entsperrfläche
- 118: Betätigungsrichtung
- 120: Widerlagerebene
- 122: Entsperrebene
- 124: Entsperrrichtung
- 126: Abstand
- 128: Halteglied
- 130: Öffnungsrichtung
- 132: Nummerierung
- 228: Entsperrvorrichtung
- 274: Entsperrkörper
- 276: Grundkörper
- 278: Entsperrkörperoberseite
- 280: Längsrichtung
- 284: Längsseite
- 285: Längsseite
- 286: Aufnahmeschlitz
- 288: Ringaufnahme
- 290: Entsperrkörperunterseite
- 308: erste Seitenfläche
- 310: zweite Seitenfläche
- 312: Entsperrelement
- 316: Entsperrfläche
- 318: Betätigungsrichtung
- 322: Entsperrebene
- 328: Halteglied
- 330: Öffnungsrichtung
- 340: Linearbewegungsführungseinrichtung
- 342: Führungselement
- 344: Führungsstab
- 346: Griffelement
- 348: Haltebügel
- 350: Neigungswinkel
- 352: Ende
- 354: Betätigungsebene
- 428: Entsperrvorrichtung
- 518: Betätigungsrichtung
- 540: Schwenkbewegungsführungseinrichtung
- 556: Schwenklager
- 558: Band
- 560: Band
- 562: Schwenkachse
- 628: Entsperrvorrichtung
- 672: Aufnahmekörper
- 674: Entsperrkörper
- 680: Längsrichtung
- 688: Ringaufnahme
- 700: erste Stirnseite
- 702: zweite Stirnseite
- 712: Entsperrelement
- 714: Widerlagerfläche
- 716: Entsperrfläche
- 718: Betätigungsrichtung
- 720: Widerlagerebene
- 722: Entsperrebene
- 746: Griffelement
- 750: Neigungswinkel
- 788: Ringaufnahme
- 828: Entsperrvorrichtung
- 874: Entsperrkörper
- 878: Entsperrkörperoberseite
- 880: Längsrichtung
- 890: Entsperrkörperunterseite
- 912: Entsperrelement
- 916: Entsperrfläche
- 918: Betätigungsrichtung
- 922: Entsperrebene
- 946: Griffelement
- 950: Neigungswinkel
- 952: Ende
- 970: Abstand

## Patentansprüche

1. Entsperrvorrichtung (28; 228; 428; 628; 828) zum Entsperren von zusammenwirkende Sperrelemente (62, 64) umfassenden Sperreinrichtungen (30) von mindestens zwei medizinischen Ringinstrumenten (26), wobei die zusammenwirkenden Sperrelemente (62, 64) in einer Sperrstellung kraft- und/oder formschlüssig in Eingriff und in einer Entsperrstellung außer Eingriff stehen, wobei die Ringinstrumente (26) jeweils eine erste Branche (32) mit einem ersten distalen Ende (36) und einem ersten proximalen Ende (38) und jeweils eine zweite Branche (34) mit einem zweiten distalen Ende (40) und einem zweiten proximalen Ende (42) umfassen, wobei die erste Branche (32) und die zweite Branche (34) jedes Ringinstruments (26) um eine Schwenkachse (44) verschwenkbar aneinander gelagert sind, wobei am ersten proximalen Ende (38) der ersten Branche (32) jedes Ringinstruments (26) ein erster Ring (54) und wobei am zweiten proximalen Ende (42) der zweiten Branche (34) jedes Ringinstruments (26) ein zweiter Ring (56) angeordnet oder ausgebildet ist, wobei jeder erste Ring (54) eine erste Ringebene (58) definiert und wobei jeder zweite Ring (56) eine zweite Ringebene (60) definiert, wobei die erste Ringebene (58) und die zweite Ringebene (60) jedes Ringinstruments (26) parallel oder im Wesentlichen parallel zueinander verlaufen, wobei die Schwenkachse (44) quer, insbesondere senkrecht, zur ersten Ringebene (58) und/oder zur zweiten Ringebene (60) verläuft, wobei die Entsperrvorrichtung (28) einen Aufnahmekörper (72; 672) mit mindestens zwei Ringaufnahmen (88; 688) zum Aufnehmen der ersten Ringe (54) der mindestens zwei Ringinstrumente (26) umfasst, wobei die Entsperrvorrichtung einen Entsperrkörper (74; 274; 674) mit mindestens zwei mit den zweiten Ringen (56) der mindestens zwei Ringinstrumenten (26) zusammenwirkenden Entsperrelementen (112; 312; 712) umfasst, wobei der Entsperrkörper (74; 274; 674) und der Aufnahmekörper (72; 672) relativ zueinander in einer Betätigungsrichtung (118; 318; 518; 718; 918) bewegbar angeordnet sind derart, dass dabei die mindestens zwei Entsperrelemente (112; 312; 712) an den zweiten Ringen (56) angreifen und diese relativ zu den ersten Ringen (54) in einer Entsperrrichtung (124) quer, insbesondere senkrecht, zur ersten Ringebene (58) und zur zweiten Ringebene (60) bewegen zum Überführen der Sperreinrichtungen (30) der mindestens zwei Ringinstrumente (26) von der Sperrstellung in die Entsperrstellung.

2. Entsperrvorrichtung nach Anspruch 1, **dadurch gekennzeichnet, dass** der Aufnahmekörper (72) und der Entsperrkörper (274) zum Führen einer Bewegung relativ zueinander gekoppelt sind.

3. Entsperrvorrichtung nach einem der voranstehenden Ansprüche, **dadurch gekennzeichnet, dass** die Entsperrvorrichtung (228; 428) eine Linearbewegungsführungseinrichtung (340) und/oder eine Schwenkbewegungsführungseinrichtung (540) umfasst,
wobei insbesondere
a) die Schwenkbewegungsführungseinrichtung (540) ein Schwenklager (556) umfasst
und/oder
b) die Linearbewegungsführungseinrichtung (340) mindestens ein geradlinig ausgebildetes Führungselement (342) umfasst, insbesondere in Form eines Führungsstabs (344),
wobei insbesondere das mindestens eine Führungselement (342) am Entsperrkörper (374) oder am Aufnahmekörper (72) angeordnet oder ausgebildet ist und in einer Führungselementaufnahme (98) am anderen Körper (72; 374) verschieblich aufgenommen ist.

4. Entsperrvorrichtung nach einem der voranstehenden Ansprüche, **dadurch gekennzeichnet, dass**
a) der Aufnahmekörper (72; 672) eine Mehrzahl von Ringaufnahmen (88; 688) umfasst, dass in jeder der Mehrzahl von Ringaufnahmen (88; 688) ein erster Ring (54) eines Ringinstruments (26) mindestens teilweise, insbesondere vollständig, einführbar ist und dass der Entsperrkörper (74; 274; 674) eine Mehrzahl von Entsperrelementen (112; 312; 712) umfasst
und/oder
b) eine Anzahl der Ringaufnahmen (88; 688) des Aufnahmekörpers (72; 672) einer Anzahl der Entsperrelemente (112; 312; 712) des Entsperrkörpers (74; 274; 674) entspricht
und/oder
c) die mindestens zwei Ringaufnahmen (88; 688) des Aufnahmekörpers (72; 672) und/oder die mindestens zwei Entsperrelemente (112; 312; 712) fortlaufend nummeriert sind
und/oder
d) jede der mindestens zwei Ringaufnahmen (88; 688) in Form eines Aufnahmeschlitzes (86) oder einer Aufnahmenut ausgebildet ist und/oder
e) jede der mindestens zwei Ringaufnahmen (88, 688) eine Widerlagerfläche (114; 714) für einen aufgenommenen ersten Ring (54) aufweist und dass die Widerlagerfläche (114; 714) parallel oder im Wesentlichen parallel zur ersten Ringebene (58) verläuft,
wobei insbesondere jede Widerlagerfläche (114; 714) eine Widerlagerebene (120; 720) definiert, dass jede Entsperrfläche (116; 716) eine Entsperrebene (122; 722) definiert und dass ein Abstand (126) zwischen der Widerlagerebene (120) und der Entsperrebene (122) von Ringaufnahmen (88) und Entsperrelementen (112), die einander zugeordnet sind zum Lösen der Sperreinrichtung (30) eines der mindestens zwei Ringinstrumente (26), beim Bewegen des Entsperrkörpers (74) und des Aufnahmekörpers (72) relativ zueinander in der Betätigungsrichtung (118) abnimmt.

5. Entsperrvorrichtung nach einem der voranstehenden Ansprüche, **dadurch gekennzeichnet, dass**
a) die mindestens zwei Entsperrelemente (112; 312; 712) jeweils eine Entsperrfläche (116; 316; 716) aufweisen, an denen die zweiten Ringe (56) bei einer Bewegung des Entsperrkörpers (74; 274; 674) und des Aufnahmekörpers (72; 672) relativ zueinander in der Betätigungsrichtung (118; 318; 518; 718; 918) anliegen und/oder aufgleiten
und/oder
b) der Aufnahmekörper (72) eine Längsdurchbrechung (82) aufweist und dass die Längsdurchbrechung (82) die mindestens zwei Ringaufnahmen (88) durchsetzt,
wobei insbesondere
- die Längsdurchbrechung (82) in Form einer Bohrung ausgebildet ist
und/oder
- die Entsperrvorrichtung (28; 228) ein Halteglied (128; 328) umfasst, welches in einer Haltestellung in der Längsdurchbrechung (82) aufgenommen ist,
wobei insbesondere das Halteglied (128; 328) stabförmig ausgebildet ist.

6. Entsperrvorrichtung nach einem der voranstehenden Ansprüche, **dadurch gekennzeichnet, dass**
a) der Aufnahmekörper (72; 672) quaderförmig oder im Wesentlichen quaderförmig ausgebildet ist und eine Längsrichtung (80; 680) definiert und dass die Längsrichtung (80; 680) quer, insbesondere senkrecht, zu den Ringaufnahmen (88; 688) verläuft,
wobei insbesondere
- die Längsrichtung (80) quer zu den Widerlagerflächen (114) verläuft
und/oder
- sich die Längsdurchbrechung (82) parallel zur Längsrichtung (80) erstreckt
und/oder
- die Betätigungsrichtung (118) parallel zur Längsrichtung (80) verläuft,
und/oder
b) der Aufnahmekörper (72) und der Entsperrkörper (74) in einer Richtung (118) quer, insbesondere senkrecht, zu den mindestens zwei Ringaufnahmen (88), insbesondere quer zu den ersten Ringebenen (58) der mindestens zwei Ringinstrumente (26), bewegbar sind zum Überführen der Sperreinrichtungen (30) der mindestens zwei Ringinstrumente (26) von der Sperrstellung in die Entsperrstellung.

7. Entsperrvorrichtung nach einem der voranstehenden Ansprüche, **dadurch gekennzeichnet, dass**
a) der Aufnahmekörper (72) und der Entsperrkörper (74) identisch ausgebildet sind,
wobei insbesondere
- die Widerlagerflächen (114) des identisch zum Aufnahmekörper (72) ausgebildeten Entsperrkörpers (74) die Entsperrflächen (116) definieren
und/oder
- die Ringaufnahmen (88) des Aufnahmekörpers (72) und die Ringaufnahmen (88) des identisch zum Aufnahmekörper (72) ausgebildeten Entsperrkörpers (74) einander spiegelsymmetrisch gegenüberliegend angeordnet sind zum Aufnehmen einerseits der ersten Ringe (54) und andererseits der zweiten Ringe (56) der mindestens zwei Ringinstrumente (26),
und/oder
b) sich die Ringaufnahmen (88) von einer Aufnahmekörperoberseite (78) zu einer Aufnahmekörperunterseite (90) erstrecken
und/oder
c) der Aufnahmekörper (72) und der Entsperrkörper (74) zum Öffnen der Ringinstrumente (26) nach dem Überführen von der Sperrstellung in die Entsperrstellung durch Verschwenken der ersten und zweiten Ringe (54, 56) voneinander weg in einer Öffnungsrichtung (130) bewegbar sind, die quer, insbesondere senkrecht, zur Betätigungsrichtung (118) verläuft.

8. Entsperrvorrichtung nach einem der Ansprüche 1 bis 6, **dadurch gekennzeichnet, dass** der Aufnahmekörper (72) und der Entsperrkörper (274) in der Betätigungsrichtung (318) parallel oder im Wesentlichen parallel zu den mindestens zwei Ringaufnahmen (88), insbesondere parallel zu den ersten Ringebenen (58) der mindestens zwei Ringinstrumente (26), bewegbar sind zum Überführen der Sperreinrichtungen (30) der mindestens zwei Ringinstrumente (26) von der Sperrstellung in die Entsperrstellung,
wobei insbesondere
a) die Betätigungsrichtung (318) in einer Betätigungsebene (354) verläuft, die sich parallel zu den ersten und/oder zweiten Ringebenen (58, 60) erstreckt,
wobei insbesondere der Aufnahmekörper (72) und der Entsperrkörper (274) relativ zueinander in der Betätigungsebene (354) verschiebbar und/oder um eine quer, insbesondere senkrecht, zu den ersten und/oder zweiten Ringebenen (58, 60) verlaufende Schwenkachse (562) verschwenkbar angeordnet oder ausgebildet sind,
und/oder
b) die Betätigungsrichtung (318) quer, insbesondere senkrecht, zur Längsrichtung (80) des Aufnahmekörpers (72) verläuft und/oder
c) die zweiten Ringe (56) bei einer Bewegung des Entsperrkörpers (274) und des Aufnahmekörpers (72) relativ zueinander in der Betätigungsrichtung (318) jeweils an einer Entsperrfläche (316) des Entsperrkörpers (274) anliegen und aufgleiten.

9. Entsperrvorrichtung nach Anspruch 8, **dadurch gekennzeichnet, dass** von den Entsperrflächen (316; 716) definierten Entsperrebenen (322; 722) relativ zu den zugeordneten Widerlagerebenen (120; 720) um einen Neigungswinkel (350; 750) geneigt sind,
wobei insbesondere
a) der Neigungswinkel (350; 750) in einem Bereich von etwa 10° bis etwa 50° liegt
und/oder
b) - der Neigungswinkel (350) aller Entsperrflächen (316) identisch ist
oder
- sich die Neigungswinkel (750) der Entsperrflächen (716) des Entsperrkörpers (674) unterscheiden,
wobei insbesondere die Neigungswinkel (750) benachbarter Entsperrflächen (716) sukzessive zunehmen, insbesondere um einen Änderungswinkel in einem Bereich von etwa 1° bis etwa 10°, insbesondere um etwa 2° bis etwa 3°.

10. Entsperrvorrichtung nach Anspruch 8 oder 9, **dadurch gekennzeichnet, dass**
a) ein Abstand von in Richtung auf den Aufnahmekörper (72) hin weisenden Enden (352) der Entsperrflächen (316) von einer Entsperrkörperunterseite (290), die in Richtung auf den Aufnahmekörper (72) hin weist, identisch sind
und/oder
b) ein Abstand (970) von in Richtung auf den Aufnahmekörper (72) hin weisenden Enden (952) der Entsperrflächen (916) von einer Entsperrkörperunterseite (890), die in Richtung auf den Aufnahmekörper (72) hin weist, unterschiedlich ist,
wobei der Abstand (970) benachbarter Entsperrflächen (916) von der Entsperrkörperunterseite (890) sukzessive zunimmt, insbesondere linear.

11. Entsperrvorrichtung nach einem der voranstehenden Ansprüche, **dadurch gekennzeichnet, dass** der Aufnahmekörper (72; 672) eine Kopplungseinrichtung umfasst zum kraft- und/oder formschlüssigen Koppeln mit einem Siebkorb (10) in einer Kopplungsstellung.

12. Entsperrvorrichtung nach einem der voranstehenden Ansprüche, **dadurch gekennzeichnet, dass**
a) die Entsperrvorrichtung (28; 228) ausgebildet ist zum gleichzeitigen Lösen aller Sperreinrichtungen (30) der in der Entsperrvorrichtung (28; 228) aufgenommenen Ringinstrumente (26) bei einer Bewegung des Entsperrkörpers (74; 274) und des Aufnahmekörpers (72) relativ zueinander in der Betätigungsrichtung (118; 318)
oder
b) die Entsperrvorrichtung (628; 828) ausgebildet ist zum sukzessiven Lösen aller Sperreinrichtungen (30) der in der Entsperrvorrichtung (628; 828) aufgenommenen Ringinstrumente (26) bei einer Bewegung des Entsperrkörpers (674; 874) und des Aufnahmekörpers (672; 72) relativ zueinander in der Betätigungsrichtung (718; 918).

13. Entsperrvorrichtung nach einem der voranstehenden Ansprüche, **dadurch gekennzeichnet, dass**
a) die Entsperrvorrichtung (28; 228; 428; 628; 828) aus einem oder mehreren Werkstoffen ausgebildet ist, die heißdampfsterilisierbar sind, insbesondere aus Polyetheretherketon und/oder einem Instrumentenstahl,
und/oder
b) am Aufnahmekörper und/oder am Entsperrkörper (274) ein Griffelement (346; 746; 946) angeordnet ist zum Handhaben der Entsperrvorrichtung (228; 628; 828).

14. Siebkorb (10) mit einem Aufnahmeraum (12) zur Aufnahme chirurgischer Instrumente, **dadurch gekennzeichnet, dass** im Aufnahmeraum (12) eine Entsperrvorrichtung (28; 228; 428; 628; 828) nach einem der voranstehenden Ansprüche angeordnet ist,
wobei insbesondere der Siebkorb einen Siebkorbboden (14) und sich vom Siebkorbboden (14) quer, insbesondere senkrecht, weg erstreckende Siebkorbwände (16, 18, 20, 22) umfasst, dass der Siebkorbboden (14) und die Siebkorbwände (16, 18, 20, 22) den Aufnahmeraum (12) begrenzen und dass der Siebkorbboden (14) und/oder die Siebkorbwände (16, 18, 20, 22) mit einer Mehrzahl von Perforationen (24) versehen sind,
wobei weiter insbesondere die Kopplungseinrichtung des Aufnahmekörpers (72; 672) ausgebildet ist zum kraft- und/oder formschlüssigen in Eingriff Bringen mit Perforationen (24) des Siebkorbbodens (14) und/oder der Siebkorbwände (16, 18, 20, 22) in der Kopplungsstellung.

15. Verwendung einer Entsperrvorrichtung (28) nach einem der Ansprüche 1 bis 13 zum gleichzeitigen oder sukzessiven außer Eingriff Bringen miteinander zusammenwirkender Sperrelemente (62, 64) von Sperreinrichtungen (30) von mindestens zwei medizinischen Ringinstrumenten (26).

## Claims

1. Unlocking apparatus (28; 228; 428; 628; 828) for unlocking locking devices (30) of at least two medical ring instruments (26), said locking devices (30) comprising cooperating locking elements (62, 64), wherein the cooperating locking elements (62, 64) are in force-locking and/or positive-locking engagement in a locked position and are out of engagement in an unlocked position, wherein the ring instruments (26) each comprise a first branch (32) with a first distal end (36) and a first proximal end (38) and each comprise a second branch (34) with a second distal end (40) and a second proximal end (42), wherein the first branch (32) and the second branch (34) of each ring instrument (26) are mounted on one another so as to be pivotable about a pivot axis (44), wherein a first ring (54) is arranged or formed on the first proximal end (38) of the first branch (32) of each ring instrument (26) and wherein a second ring (56) is arranged or formed on the second proximal end (42) of the second branch (34) of each ring instrument (26), wherein each first ring (54) defines a first ring plane (58) and wherein each second ring (56) defines a second ring plane (60), wherein the first ring plane (58) and the second ring plane (60) of each ring instrument (26) run in parallel or substantially in parallel to one another, wherein the pivot axis (44) runs transversely, in particular perpendicularly, to the first ring plane (58) and/or to the second ring plane (60), wherein the unlocking apparatus (28) comprises a receiving body (72; 672) with at least two ring receptacles (88; 688) for accommodating the first rings (54) of the at least two ring instruments (26), wherein the unlocking apparatus comprises an unlocking body (74; 274; 674) with at least two unlocking elements (112; 312; 712) cooperating with the second rings (56) of the at least two ring instruments (26), wherein the unlocking body (74; 274; 674) and the receiving body (72; 672) are arranged so as to be movable relative to one another in an actuating direction (118; 318; 518; 718; 918) in such a way that the at least two unlocking elements (112; 312; 712) thereby engage on the second rings (56) and move same relative to the first rings (54) in an unlocking direction (124) transversely, in particular perpendicularly, to the first ring plane (58) and to the second ring plane (60) for transferring the locking devices (30) of the at least two ring instruments (26) from the locked position into the unlocked position.

2. Unlocking apparatus in accordance with Claim 1, **characterized in that** the receiving body (72) and the unlocking body (274) are coupled for guiding a movement relative to one another.

3. Unlocking apparatus in accordance with any one of the preceding Claims, **characterized in that** the unlocking apparatus (228; 428) comprises a linear movement guidance device (340) and/or a pivotal movement guidance device (540),
wherein, in particular,
a) the pivotal movement guidance device (540) comprises a pivot bearing (556)
and/or
b) the linear movement guidance device (340) comprises at least one rectilinearly configured guidance element (342), in particular in the form of a guide rod (344),
wherein, in particular, the at least one guidance element (342) is arranged or formed on the unlocking body (374) or on the receiving body (72) and is displaceably accommodated in a guidance element receptacle (98) on the other body (72; 374).

4. Unlocking apparatus in accordance with any one of the preceding Claims, **characterized in that**
a) the receiving body (72; 672) comprises a plurality of ring receptacles (88; 688), **in that** a first ring (54) of a ring instrument (26) is insertable at least partially, in particular completely, in each of the plurality of ring receptacles (88; 688), and **in that** the unlocking body (74; 274; 674) comprises a plurality of unlocking elements (112; 312; 712)
and/or
b) a number of ring receptacles (88; 688) of the receiving body (72; 672) corresponds to a number of unlocking elements (112; 312; 712) of the unlocking body (74; 274; 674)
and/or
c) the at least two ring receptacles (88; 688) of the receiving body (72; 672) and/or the at least two unlocking elements (112; 312; 712) are consecutively numbered
and/or
d) each of the at least two ring receptacles (88; 688) is configured in the form of a receiving slot (86) or a receiving groove
and/or
e) each of the at least two ring receptacles (88; 688) has an abutment surface (114; 714) for an accommodated first ring (54) and **in that** the abutment surface (114; 714) runs in parallel or substantially in parallel to the first ring plane (58),
wherein, in particular, each abutment surface (114; 714) defines an abutment plane (120; 720), **in that** each unlocking face (116; 716) defines an unlocking plane (122; 722), and **in that** a distance (126) between the abutment plane (120) and the unlocking plane (122) of ring receptacles (88) and unlocking elements (112), which are associated with one another for releasing the locking device (30) of one of the at least two ring instruments (26), decreases upon moving the unlocking body (74) and the receiving body (72) relative to one another in the actuating direction (118).

5. Unlocking apparatus in accordance with any one of the preceding Claims, **characterized in that**
a) the at least two unlocking elements (112; 312; 712) each have an unlocking face (116; 316; 716) on which the second rings (56) abut and/or slide upon a movement of the unlocking body (74; 274; 674) and the receiving body (72; 672) relative to one another in the actuating direction (118; 318; 518; 718; 918)
and/or
b) the receiving body (72) has a longitudinal perforation (82) and **in that** the longitudinal perforation (82) passes through the at least two ring receptacles (88),
wherein, in particular,
- the longitudinal perforation (82) is configured in the form of a bore
and/or
- the unlocking apparatus (28; 228) comprises a holding member (128; 328), which is accommodated in the longitudinal perforation (82) in a holding position,
wherein, in particular, the holding member (128; 328) is of rod-shaped configuration.

6. Unlocking apparatus in accordance with any one of the preceding Claims, **characterized in that**
a) the receiving body (72; 672) is of cuboidal or substantially cuboidal configuration and defines a longitudinal direction (80; 680) and **in that** the longitudinal direction (80; 680) runs transversely, in particular perpendicularly, to the ring receptacles (88; 688), wherein, in particular,
- the longitudinal direction (80) runs transversely to the abutment surfaces (114)
and/or
- the longitudinal perforation (82) extends in parallel to the longitudinal direction (80)
and/or
- the actuating direction (118) runs in parallel to the longitudinal direction (80),
and/or
b) the receiving body (72) and the unlocking body (74) are movable in a direction (118) transverse, in particular perpendicular, to the at least two ring receptacles (88), in particular transverse to the first ring planes (58) of the at least two ring instruments (26), for transferring the locking devices (30) of the at least two ring instruments (26) from the locked position into the unlocked position.

7. Unlocking apparatus in accordance with any one of the preceding Claims, **characterized in that**
a) the receiving body (72) and the unlocking body (74) are of identical configuration,
wherein, in particular,
- the abutment surfaces (114) of the unlocking body (74) configured identically to the receiving body (72) define the unlocking faces (116)
and/or
- the ring receptacles (88) of the receiving body (72) and the ring receptacles (88) of the unlocking body (74) configured identically to the receiving body (72) are arranged mirror-symmetrically opposite one another for accommodating the first rings (54) of the at least two ring instruments (26) on the one hand and the second rings (56) of the at least two ring instruments (26) on the other hand,
and/or
b) the ring receptacles (88) extend from a receiving body top side (78) to a receiving body bottom side (90)
and/or
c) the receiving body (72) and the unlocking body (74) are movable in an opening direction (130) for opening the ring instruments (26) after transferring from the locked position into the unlocked position by pivoting the first and second rings (54, 56) away from one another, said opening direction (130) running transversely, in particular perpendicularly, to the actuating direction (118).

8. Unlocking apparatus in accordance with any one of Claims 1 to 6, **characterized in that** the receiving body (72) and the unlocking body (274) are movable in the actuating direction (318) in parallel or substantially in parallel to the at least two ring receptacles (88), in particular in parallel to the first ring planes (58) of the at least two ring instruments (26), for transferring the locking devices (30) of the at least two ring instruments (26) from the locked position into the unlocked position,
wherein, in particular,
a) the actuating direction (318) runs in an actuating plane (354) that extends in parallel to the first and/or second ring planes (58, 60), wherein, in particular, the receiving body (72) and the unlocking body (274) are arranged or formed so as to be displaceable relative to one another in the actuating plane (354) and/or pivotable about a pivot axis (562) running transversely, in particular perpendicularly, to the first and/or second ring planes (58, 60),
and/or
b) the actuating device (318) runs transversely, in particular perpendicularly, to the longitudinal direction (80) of the receiving body (72),
and/or
c) the second rings (56) each abut against and slide on an unlocking face (316) of the unlocking body (274) upon a movement of the unlocking body (274) and the receiving body (72) relative to one another in the actuating direction (318).

9. Unlocking apparatus in accordance with Claim 8, **characterized in that** unlocking planes (322; 722) defined by the unlocking faces (316; 716) are inclined by an angle of inclination (350; 750) relative to the associated abutment planes (120; 720),
wherein, in particular,
a) the angle of inclination (350; 750) is in a range of about 10° to about 50°
and/or
b) - the angle of inclination (350) of all unlocking faces (316) is identical
or
- the angles of inclination (750) of the unlocking faces (716) of the unlocking body (674) are different,
wherein, in particular, the angles of inclination (750) of adjacent unlocking faces (716) successively increase, in particular by an angle of change in a range of about 1° to about 10°, in particular by about 2° to about 3°.

10. Unlocking apparatus in accordance with Claim 8 or 9, **characterized in that**
a) a distance of ends (352) of the unlocking faces (316) pointing in the direction toward the receiving body (72) from an unlocking body bottom side (290), which points in the direction toward the receiving body (72), is identical
and/or
b) a distance (970) of ends (952) of the unlocking faces (916) pointing in the direction toward the receiving body (72) from an unlocking body bottom side (890), which points in the direction toward the receiving body (72), is different,
wherein the distance (970) of adjacent unlocking faces (916) from the unlocking body bottom side (890) successively increases, in particular linearly.

11. Unlocking apparatus in accordance with any one of the preceding Claims, **characterized in that** the receiving body (72; 672) comprises a coupling device for coupling to a sieve basket (10) in a force-locking and/or positive-locking manner in a coupling position.

12. Unlocking apparatus in accordance with any one of the preceding Claims, **characterized in that**
a) the unlocking apparatus (28; 228) is configured to simultaneously release all locking devices (30) of the ring instruments (26) accommodated in the unlocking apparatus (28; 228) upon a movement of the unlocking body (74; 274) and the receiving body (72) relative to one another in the actuating direction (118; 318)
or
b) the unlocking apparatus (628; 828) is configured to successively release all locking devices (30) of the ring instruments (26) accommodated in the unlocking apparatus (628; 828) upon a movement of the unlocking body (674; 874) and the receiving body (672; 72) relative to one another in the actuating direction (718; 918).

13. Unlocking apparatus in accordance with any one of the preceding Claims, **characterized in that**
a) the unlocking apparatus (28; 228; 428; 628; 828) is made of one or more materials that are sterilizable with hot steam, in particular polyetheretherketone and/or an instrument steel,
and/or
b) a grip element (346; 746; 946) is arranged on the receiving body and/or on the unlocking body (274) for handling the unlocking apparatus (228; 628; 828).

14. Sieve basket (10) with a receiving space (12) for accommodating surgical instruments, **characterized in that** an unlocking apparatus (28; 228; 428; 628; 828) in accordance with any one of the preceding Claims is arranged in the receiving space (12),
wherein, in particular, the sieve basket comprises a sieve basket base (14) and sieve basket walls (16, 18, 20, 22) extending transversely, in particular perpendicularly, away from the sieve basket base (14), **in that** the sieve basket base (14) and the sieve basket walls (16, 18, 20, 22) delimit the receiving space (12), and **in that** the sieve basket base (14) and/or the sieve basket walls (16, 18, 20, 22) are provided with a plurality of perforations (24),
wherein, further in particular, the coupling device of the receiving body (72; 672) is configured to be brought into force-locking and/or positive-locking engagement with perforations (24) of the sieve basket base (14) and/or of the sieve basket walls (16, 18, 20, 22) in the coupling position.

15. Use of an unlocking apparatus (28) in accordance with any one of Claims 1 to 13 for simultaneously or successively bringing cooperating locking elements (62, 64) of locking devices (30) of at least two medical ring instruments (26) out of engagement.

## Revendications

1. Dispositif de déverrouillage (20; 228; 428; 628; 828) pour déverrouiller des dispositifs de verrouillage (30) comprenant des éléments de verrouillage coopérants (62, 64) d'au moins deux instruments à anneaux médicaux (26), où les éléments de verrouillage coopérants (62, 64) sont en prise par assemblage de force et/ou de forme dans une position de verrouillage et sont hors de prise dans une position de déverrouillage, où les instruments à anneaux (26) comprennent à chaque fois une première branche (32) avec une première extrémité distale (36) et une première extrémité proximale (38) et à chaque fois une deuxième branche (34) avec une deuxième extrémité distale (40) et une deuxième extrémité proximale (42), où la première branche (32) et la deuxième branche (34) de chaque instrument à anneaux (26) sont montées de manière pivotante l'une contre l'autre autour d'un axe de pivotement (44), où à la première extrémité proximale (38) de la première branche (32) de chaque instrument à anneaux (26) est disposé ou agencé un premier anneau (54) et où à la deuxième extrémité proximale (42) de la deuxième branche (34) de chaque instrument à anneaux (26) est disposé ou agencé un deuxième anneau (56), où chaque premier anneau (54) définit un premier plan d'anneau (58) et où chaque deuxième anneau (56) définit un deuxième plan d'anneau (60), où le premier plan d'anneau (58) et le deuxième plan d'anneau (60) de chaque instrument à anneaux (26) s'étendent parallèlement ou sensiblement parallèlement l'un à l'autre, où l'axe de pivotement (44) s'étend transversalement, en particulier perpendiculairement, au premier plan d'anneau (58) et/ou au deuxième plan d'anneau (60), où le dispositif de déverrouillage (28) comprend un corps de logement (72; 672) avec au moins deux logements d'anneau (88; 688) pour recevoir les premiers anneaux (54) des au moins deux instruments à anneaux (26), où le dispositif de déverrouillage comprend un corps de déverrouillage (74; 274; 674) avec au moins deux éléments de déverrouillage (112; 312; 712) coopérant avec les deuxièmes anneaux (56) des au moins deux instruments à anneaux (26), où le corps de déverrouillage (74; 274; 674) et le corps de logement (72; 672) sont disposés mobiles l'un par rapport à l'autre dans une direction d'actionnement (118; 318; 518; 718; 918) de telle sorte que les au moins deux éléments de déverrouillage (112; 312; 712) agissent sur les deuxièmes anneaux (56) et déplacent ceux-ci par rapport aux premiers anneaux (54) dans une direction de déverrouillage (124) transversalement, en particulier perpendiculairement, au premier plan d'anneau (58) et au deuxième plan d'anneau (60) pour faire passer les dispositifs de verrouillage (30) des au moins deux instruments à anneaux (26) de la position de verrouillage à la position de déverrouillage.

2. Dispositif de déverrouillage selon la revendication 1, **caractérisé en ce que** le corps de logement (72) et le corps de déverrouillage (274) sont couplés l'un à l'autre pour le guidage d'un mouvement.

3. Dispositif de déverrouillage selon l'une des revendications précédentes, **caractérisé en ce que** le dispositif de déverrouillage (228; 428) comprend un dispositif de guidage de mouvement linéaire (340) et/ou un dispositif de guidage de mouvement de pivotement (540),
où en particulier
a) le dispositif de guidage de mouvement de pivotement (540) comprend un palier pivotant (556)
et /ou
b) le dispositif de guidage de mouvement linéaire (340) comprend au moins un élément de guidage agencé de manière rectiligne (342), en particulier sous forme d'une barre de guidage (344),
où en particulier le au moins un élément de guidage (342) est disposé ou agencé sur le corps de déverrouillage (374) ou sur le corps de logement (72) et est logé de manière coulissante dans un logement d'élément de guidage (98) sur l'autre corps (72; 374).

4. Dispositif de déverrouillage selon l'une des revendications précédentes, **caractérisé en ce que**
a) le corps de logement (72; 672) comprend une pluralité de logements d'anneau (88; 688), **en ce que** dans chacun de la pluralité de logements d'anneau (88; 688) un premier anneau (54) d'un instrument à anneaux (26) peut être inséré au moins partiellement, en particulier entièrement, et **en ce que** le corps de déverrouillage (74; 274; 674) comprend une pluralité d'éléments de déverrouillage (112; 312; 712),
et /ou
b) un certain nombre de logements d'anneau (88; 688) du corps de logement (72; 672) correspond à un certain nombre d'éléments de déverrouillage (112; 312; 712) du corps de déverrouillage (74; 274; 674),
et/ou
c) les au moins deux logements d'anneau (88; 688) du corps de logement (72; 672) et/ou les au moins deux éléments de déverrouillage (112; 312; 712) sont numérotés consécutivement
et/ ou
d) chacun des au moins deux logements d'anneau (88; 688) est agencé sous la forme d'une fente de logement (86) ou d'une rainure de logement
et/ou
e) chacun des au moins deux logements d'anneau (88, 688) présente une surface de butée (114; 714) pour un premier anneau (54) reçu et **en ce que** la surface de butée (114; 714) s'étend parallèlement ou sensiblement parallèlement au premier plan d'anneau (58), où, en particulier, chaque surface de butée (114; 714) définit un plan de butée (120; 720), **en ce que** chaque surface de déverrouillage (116; 716) définit un plan de déverrouillage (122; 722) et **en ce qu'**une distance (126) entre le plan de butée (120) et le plan de déverrouillage (122) de logements d'anneau (88) et d'éléments de déverrouillage (112) qui sont affectés les uns aux autres pour libérer le dispositif de verrouillage (30) de l'un des au moins deux instruments à anneaux (26), diminue lors du mouvement du corps de déverrouillage (74) et du corps de logement (72) l'un par rapport à l'autre dans la direction d'actionnement (118).

5. Dispositif de déverrouillage selon l'une des revendications précédentes, **caractérisé en ce que**
a) les au moins deux éléments de déverrouillage (112; 312; 712) présentent à chaque fois une surface de déverrouillage (116; 316; 716) sur laquelle les deuxièmes anneaux (56) s'appuient et/ou glissent lors d'un mouvement du corps de déverrouillage (74; 274; 674) et du corps de logement (72; 672) l'un par rapport à l'autre dans la direction d'actionnement (118; 318; 518; 718; 918)
et/ou
b) le corps de logement (72) présente une ouverture longitudinale (82) et **en ce que** l'ouverture longitudinale (82) traverse les au moins deux logements d'anneau (88),
où en particulier
- l'ouverture longitudinale (82) est agencée sous forme d'un alésage
et/ou
- le dispositif de déverrouillage (28; 228) comprend un organe de maintien (128; 328) qui est logé dans une position de maintien dans l'ouverture longitudinale (82),
où en particulier l'élément de maintien (128; 328) est agencé sous forme de tige.

6. Dispositif de déverrouillage selon l'une des revendications précédentes, **caractérisé en ce que**
a) le corps de logement (72; 672) est agencé sous forme de parallélépipède ou sensiblement sous forme de parallélépipède et définit une direction longitudinale (80; 680) et **en ce que** la direction longitudinale (80; 680) s'étend transversalement, en particulier perpendiculairement, aux logements d'anneau (88; 688),
où en particulier
- la direction longitudinale (80) s'étend transversalement aux surfaces de butée (114)
et/ou
- l'ouverture longitudinale (82) s'étend parallèlement à la direction longitudinale (80)
et/ou
- la direction d'actionnement (118) s'étend parallèlement à la direction longitudinale (80),
et/ou
b) le corps de logement (72) et le corps de déverrouillage (74) sont mobiles dans une direction (118) transversalement, en particulier perpendiculairement, aux au moins deux logements d'anneau (88), en particulier transversalement aux premiers plans d'anneau (58) des au moins deux instruments à anneaux (26) pour faire passer les dispositifs de verrouillage (30) des au moins deux instruments à anneaux (26) de la position de verrouillage à la position de déverrouillage.

7. Dispositif de déverrouillage selon l'une des revendications précédentes, **caractérisé en ce que**
a) le corps de logement (72) et le corps de déverrouillage (74) sont agencés de manière identique,
où en particulier
- les surfaces de butée (114) du corps de déverrouillage (74) agencé de manière identique au corps de logement (72) définissent les surfaces de déverrouillage (116)
et/ou
- les logements d'anneau (88) du corps de logement (72) et les logements d'anneau (88) du corps de déverrouillage (74) agencé de manière identique au corps de logement (72) sont disposés de manière symétrique dans un miroir les uns par rapport aux autres pour loger d'une part les premiers anneaux (54) et d'autre part les deuxièmes anneaux (56) des au moins deux instruments à anneaux (26),
et/ou
b) les logements d'anneau (88) s'étendent d'un côté supérieur de corps de logement (78) à un côté inférieur de corps de logement (90)
et/ou
c) le corps de logement (72) et le corps de déverrouillage (74) sont mobiles l'un par rapport à l'autre pour l'ouverture des instruments à anneaux (26) après le passage de la position de verrouillage à la position de déverrouillage par pivotement des premiers et deuxièmes anneaux (54, 56) en s'éloignant les uns des autres dans une direction d'ouverture (130), qui s'étend transversalement, en particulier perpendiculairement, à la direction d'actionnement (118).

8. Dispositif de déverrouillage selon l'une des revendications 1 à 6, **caractérisé en ce que** le corps de logement (72) et le corps de déverrouillage (274) sont mobiles dans la direction d'actionnement (318) parallèlement ou sensiblement parallèlement aux au moins deux logements d'anneau (88), en particulier parallèlement aux premiers plans d'anneau (58) des au moins deux instruments à anneaux (26), pour le passage des dispositifs de verrouillage (30) des au moins deux instruments à anneaux (26) de la position de verrouillage à la position de déverrouillage,
où en particulier
a) la direction d'actionnement (318) s'étend dans un plan d'actionnement (354) qui s'étend parallèlement aux premiers et/ou deuxièmes plans d'anneau (58, 60),
où en particulier le corps de logement (72) et le corps de déverrouillage (274) sont agencés ou formés déplaçables l'un par rapport à l'autre dans le plan d'actionnement (354) et/ou pivotants autour d'un axe de pivotement (562) s'étendant transversalement, en particulier perpendiculairement, aux premiers et/ou deuxièmes plans d'anneau (58, 60),
et/ou
b) la direction d'actionnement (318) s'étend transversalement, en particulier perpendiculairement, à la direction longitudinale (80) du corps de logement (72),
et/ou
c) les deuxièmes anneaux (56) lors d'un mouvement du corps de déverrouillage (274) et du corps de logement (72) l'un par rapport à l'autre dans la direction d'actionnement (318) butent ou glissent à chaque fois sur une surface de déverrouillage (316) du corps de déverrouillage (274).

9. Dispositif de déverrouillage selon la revendication 8, **caractérisé en ce que** les plans de déverrouillage (322; 722) définis par les surfaces de déverrouillage (316; 716) sont inclinés par rapport aux plans de butée (120; 720) associés d'un angle d'inclinaison (350; 750),
où en particulier
a) l'angle d'inclinaison (350; 750) est situé dans un domaine d'environ 10° à environ 50°
et/ou
b) - l'angle d'inclinaison (350) de toutes les surfaces de déverrouillage (316) est identique
ou
- l'angle d'inclinaison (750) des surfaces de déverrouillage (716) du corps de déverrouillage (674) diffèrent,
où en particulier, les angles d'inclinaison (750) de surfaces de déverrouillage (716) voisines augmentent successivement, en particulier d'un angle de changement dans un domaine d'environ 1° à environ 10°, en particulier d'environ 2° à environ 3°.

10. Dispositif de déverrouillage selon la revendication 8 ou 9, **caractérisé en ce que**
a) une distance d'extrémités (352) des surfaces de déverrouillage (316) tournées dans la direction du corps de logement (72) d'un côté inférieur de corps de déverrouillage (290) qui est tourné dans la direction du corps de logement (72), est identique
et/ou
b) une distance (970) d'extrémités (952) des surfaces de déverrouillage (916) tournées vers le corps de logement (72) d'un côté inférieur de corps de déverrouillage (890), qui est tourné dans la direction du corps de logement (72), est variable,
où la distance (970) de surfaces de déverrouillage (916) voisines du côté inférieur du corps de déverrouillage (890) augmente progressivement, en particulier linéairement.

11. Dispositif de déverrouillage selon l'une des revendications précédentes, **caractérisé en ce que** le corps de logement (72; 672) comprend une unité de couplage pour le couplage par assemblage de force et/ou de forme avec un panier criblé (10) dans une position de couplage.

12. Dispositif de déverrouillage selon l'une des revendications précédentes, **caractérisé en ce que**
a) le dispositif de déverrouillage (28; 228) est conçu pour la libération simultanée de tous les dispositifs de verrouillage (30) des instruments à anneaux (26) logés dans le dispositif de déverrouillage (28; 228) lors d'un mouvement du corps de déverrouillage (74; 274) et du corps de logement (72) l'un par rapport à l'autre dans la direction d'actionnement (118; 318)
ou
b) le dispositif de déverrouillage (628; 828) est agencé pour la libération successive de tous les dispositifs de verrouillage (30) des instruments à anneaux (26) logés dans le dispositif de déverrouillage (628; 828) lors d'un mouvement du corps de déverrouillage (674; 874) et du corps de logement (672; 72) l'un par rapport à l'autre dans la direction d'actionnement (718; 918).

13. Dispositif de déverrouillage selon l'une des revendications précédentes, **caractérisé en ce que**
a) le dispositif de déverrouillage (28; 228; 428; 628; 828) est agencé en un ou plusieurs matériaux qui peuvent être stérilisés à la vapeur surchauffée, en particulier en polyéther-éthercétone et/ou en un acier à instruments,
et/ou
b) sur le corps de logement et/ou sur le corps de déverrouillage (274) est agencé un élément de préhension (346; 746; 946) pour la manipulation du dispositif de verrouillage (228; 628; 828).

14. Panier criblé (10) avec un espace de logement (12) pour loger des instruments chirurgicaux, **caractérisé en ce qu'**un dispositif de
déverrouillage (28; 228; 428; 628; 828) selon l'une des revendications précédentes est agencé dans l'espace de logement (12), où en particulier le panier criblé comprend un fond de panier criblé (14) et des parois de panier criblé (16, 18, 20, 22) s'écartant transversalement, en particulier perpendiculairement, du fond de panier criblé (14), **en ce que** le fond de panier criblé (14) et les parois de panier criblé (16, 18, 20, 22) délimitent l'espace de logement (12) et **en ce que** le fond de panier criblé (14) et/ou les parois de panier criblé (16, 18, 20, 22) sont munis d'une pluralité de perforations (24),
où en outre en particulier le dispositif de couplage du corps de logement (72; 672) est agencé pour la mise en prise par assemblage de force et/ou de forme avec des perforations (24) du fond de panier criblé (14) et/ou des parois de panier criblé (16, 18, 20, 22) dans la position de couplage.

15. Utilisation d'un dispositif de déverrouillage (28) selon l'une des revendications 1 à 13 pour la mise hors de prise simultanée ou successive d'éléments de verrouillage (62, 64) coopérant entre eux de dispositifs de verrouillage (30) d'au moins deux instruments à anneaux médicaux (26).
